(19) **Europäisches Patentamt** / **European Patent Office** / **Office européen des brevets**

(11) **EP 4 640 929 A1**

(12) **EUROPEAN PATENT APPLICATION**

(43) Date of publication:
29.10.2025 Bulletin 2025/44

(21) Application number: 25171581.9

(22) Date of filing: 22.04.2025

(51) International Patent Classification (IPC):
C30B 1/02 (2006.01)      C30B 29/32 (2006.01)
C04B 35/468 (2006.01)     H10N 30/00 (2023.01)
C30B 33/02 (2006.01)

(52) Cooperative Patent Classification (CPC):
C30B 29/32; C04B 35/4682; C04B 35/49;
C30B 1/026; C30B 33/02; H10N 30/00;
C04B 2235/3203; C04B 2235/3232;
C04B 2235/3244; C04B 2235/3263;
C04B 2235/3298; C04B 2235/768; C04B 2235/79

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 22.04.2024  JP 2024069138
28.02.2025  JP 2025031362

(71) Applicant: CANON KABUSHIKI KAISHA
Tokyo 146-8501 (JP)

(72) Inventors:
• FURUTA, Tatsuo
  Tokyo, 146-8501 (JP)
• MATSUDA, Takanori
  Tokyo, 146-8501 (JP)

(74) Representative: TBK
Bavariaring 4-6
80336 München (DE)

(54) **METHOD FOR MANUFACTURING PEROVSKITE-TYPE SINGLE CRYSTAL, PEROVSKITE-TYPE SINGLE CRYSTAL, PIEZOELECTRIC ELEMENT, ULTRASONIC MOTOR, OPTICAL DEVICE, VIBRATION DEVICE, DUST REMOVAL DEVICE, IMAGING DEVICE, ULTRASONIC PROBE, ULTRASONIC DIAGNOSIS APPARATUS, ULTRASONIC DIAGNOSIS SYSTEM AND ELECTRONIC DEVICE**

(57)    A single crystal that exhibits a high electromechanical coupling factor and a high coercive field when being used for a piezoelectric element, and a method for manufacturing the single crystal are provided. The method is a method for manufacturing a perovskite-type single crystal, wherein, through step (1) of firing a raw material containing an acceptor under an atmospheric environment to produce a first single crystal of perovskite type, step (2) including firing the first single crystal under a reducing environment, and step (3) including firing the single crystal produced in step (2) under an atmospheric environment in this order, a perovskite-type single crystal having a higher coercive field value than the first single crystal is produced.

FIG. 1

EP 4 640 929 A1

**Description**

BACKGROUND OF THE INVENTION

Field of the Invention

The present invention relates to a method for manufacturing a perovskite-type single crystal and to a perovskite-type single crystal. The present invention also relates to a piezoelectric element, an ultrasonic motor, an optical device, a vibration device, a dust removal device, an imaging device, an ultrasonic probe, an ultrasonic diagnosis apparatus, an ultrasonic diagnosis system and an electronic device which use the single crystal.

Description of the Related Art

High performance and size reduction have been promoted for piezoelectric devices in recent years, and piezoelectric materials are required to have higher piezoelectric performance than ever. A method of single crystal formation with alignment of crystal orientation in a piezoelectric material is known as a means for achieving enhanced piezoelectric performance in piezoelectric materials. If a piezoelectric material is formed as a single crystal, a high electromechanical coupling factor is obtained, and enhanced piezoelectric performance results.

For example, Japanese Patent No. 3507821 discloses a solid-phase method for single crystal formation of a piezoelectric material such as barium titanate, in which a single crystal to serve as a seed is joined to a matrix and heat-treated to cause the growth of an abnormal grain as the enlargement only of a single crystal grain, and the abnormal grain is cut out to yield a single crystal.

While the piezoelectric material obtained in the manufacturing method of Japanese Patent No. 3507821 has an enhanced electromechanical coupling factor through the single crystal formation, the piezoelectric material suffers from reduction in the coercive field, which indicates the intensity of an external electric field at which zero polarization is attained. If a piezoelectric material with a low coercive field is driven at a high voltage, the piezoelectric material disadvantageously loses piezoelectric performance.

SUMMARY OF THE INVENTION

Single crystal formation solely by means of a conventional technique as described above yields high piezoelectric characteristics, but disadvantageously causes a problem of reduction in the coercive field.

The present invention has been made to solve such a problem, and an object of the present invention is to provide a method for manufacturing a single crystal that exhibits a high electromechanical coupling factor and a high coercive field when being used for a piezoelectric element. In addition, another object of the present invention is to provide an ultrasonic motor, an optical device, a vibration device, a dust removal device, an imaging device, an ultrasonic probe, an ultrasonic diagnosis apparatus, an ultrasonic diagnosis system and an electronic device which use a piezoelectric element having a high electromechanical coupling factor and a high coercive field.

To solve the above problem, the present invention provides a method for manufacturing a perovskite-type single crystal, wherein, through step (1) of firing a raw material containing an acceptor under an atmospheric environment to produce a first single crystal of perovskite type, step (2) including firing the first single crystal under a reducing environment, and step (3) including firing the single crystal produced in step (2) under an atmospheric environment in this order, a single crystal having a higher coercive field value than the first single crystal is produced.

The present invention also provides a perovskite-type single crystal containing an oxide containing Ba, Ti and Zr with perovskite structure, and Mn, with x as the mole ratio of Zr to the sum total of Ti and Zr satisfying $0.02 \leq x \leq 0.13$, wherein the single crystal has a Mn content of 0.04 parts by mass or more and 0.36 parts by mass or less in terms of metal to 100 parts by mass of the oxide, the single crystal optionally contains Bi, and has a Bi content of 0 parts by mass or more and 0.20 parts by mass or less in terms of metal to 100 parts by mass of the oxide, and the single crystal has an electromechanical coupling factor $k_{33}$ of 80% or more at 25°C and a coercive field of 2.5 kV/cm or more.

The present invention provides a piezoelectric element including a plurality of electrodes and the single crystal.

The present invention provides an ultrasonic motor including: a vibration unit provided with the piezoelectric element; and a mobile unit in contact with the vibration unit.

The present invention provides an optical device including the ultrasonic motor in a drive section.

The present invention provides a vibration device including a vibration unit with a diaphragm provided with the piezoelectric element.

The present invention provides a dust removal device including the vibration device in a vibration section.

The present invention provides an imaging device including the dust removal device and an imaging element unit, wherein the diaphragm of the dust removal device is provided on the light-receiving-face side of the imaging element unit.

The present invention provides an ultrasonic probe including the piezoelectric element, wherein the ultrasonic probe transmits and receives ultrasonic waves by the piezoelectric element.

The present invention provides an ultrasonic diagnosis apparatus including the ultrasonic probe and an image output section.

The present invention provides an ultrasonic diagnosis system including: the ultrasonic probe; a transmitting section that transmits signals outputted from the ultrasonic probe; and a receiving section that receives signals transmitted from the transmitting section.

The present invention provides an electronic device provided with a piezoelectric acoustic part including the piezoelectric element.

Further features of the present invention will become apparent from the following description of exemplary embodiments with reference to the attached drawings.

BRIEF DESCRIPTION OF THE DRAWINGS

FIG. 1 is a schematic diagram for describing the first embodiment of the present invention.
FIG. 2 is a schematic diagram for describing the first embodiment of the present invention.
FIG. 3 is a schematic diagram for describing the first embodiment of the present invention.
FIG. 4 is a schematic diagram for describing the first embodiment of the present invention.
FIG. 5 is a schematic diagram for describing the first embodiment of the present invention.
FIG. 6 is a schematic diagram for describing the first embodiment of the present invention.
FIG. 7 is a schematic diagram for describing the first embodiment of the present invention.
FIG. 8 is a schematic diagram illustrating a mode of the configuration of the piezoelectric element of the present invention.
FIG. 9 is a schematic diagram illustrating a mode of the configuration of the ultrasonic motor of the present invention.
FIG. 10A is a schematic diagram illustrating a mode of the optical device of the present invention.
FIG. 10B is a schematic diagram illustrating another mode of the optical device of the present invention.
FIG. 11 is a schematic diagram illustrating a mode of the optical device of the present invention.
FIG. 12A is a schematic diagram illustrating a mode of the vibration device of the present invention when being used for a dust removal device.
FIG. 12B is a schematic diagram illustrating another mode of the vibration device of the present invention when being used for a dust removal device.
FIG. 13A is a schematic diagram illustrating an example of the configuration of a piezoelectric element in the dust removal device of the present invention.
FIG. 13B is a schematic diagram illustrating another example of the configuration of a piezoelectric element in the dust removal device of the present invention.
FIG. 13C is a schematic diagram illustrating another example of the configuration of a piezoelectric element in the dust removal device of the present invention.
FIG. 14A is a schematic diagram illustrating an example of the principle of the vibration of the dust removal device of the present invention.
FIG. 14B is a schematic diagram illustrating another example of the principle of the vibration of the dust removal device of the present invention.
FIG. 15 is a schematic diagram illustrating a mode of the imaging device of the present invention.
FIG. 16 is a schematic diagram illustrating a mode of the imaging device of the present invention.
FIG. 17 is a schematic diagram illustrating a mode of the ultrasonic probe of the present invention.
FIG. 18 is a schematic diagram illustrating a mode of the ultrasonic diagnosis apparatus of the present invention.
FIG. 19 is a schematic diagram illustrating a mode of the ultrasonic diagnosis system of the present invention.
FIG. 20 is a schematic diagram illustrating a mode of the ultrasonic diagnosis system of the present invention.
FIG. 21 is a schematic diagram illustrating a mode of the electronic device of the present invention.
FIG. 22 is a schematic diagram for describing an embodiment or mode of X-ray diffraction pole figure measurement for the single crystal of the present invention.
FIG. 23 is a schematic diagram for describing an embodiment or mode of X-ray diffraction pole figure measurement for the single crystal of the present invention.
FIG. 24 is a schematic diagram for describing an embodiment or mode of a PE hysteresis curve for a piezoelectric body element with the single crystal of the present invention.
FIG. 25 is a schematic diagram for describing an embodiment or mode of the temperature dependence of the piezoelectric constant of a piezoelectric body element with the single crystal of the present invention.

FIG. 26 is a schematic diagram for describing an embodiment or mode of the temperature dependence of the relative dielectric constant of a piezoelectric body element with the single crystal of the present invention.

## DESCRIPTION OF THE EMBODIMENTS

Hereinafter, embodiments for implementation of the present invention will be described.

The present invention provides a method for manufacturing a perovskite-type single crystal having a high electromechanical coupling factor and a high coercive field, and a perovskite-type single crystal produced by the method.

The perovskite-type single crystal of the present invention can be used for piezoelectric elements in various applications such as electronic devices including semiconductors, light-emitting elements, optical elements, energy conversion elements and sensors.

<First Embodiment>

The first embodiment relates to a method for manufacturing a perovskite-type single crystal.

The method of the present invention for manufacturing a perovskite-type single crystal is characterized in that, through step (1) of firing a raw material containing an acceptor under an atmospheric environment to produce a first single crystal of perovskite type, step (2) including firing the first single crystal under a reducing environment, and step (3) including firing the single crystal produced in step (2) under an atmospheric environment in this order, a single crystal having a higher coercive field value than the first single crystal is produced.

The following describes fundamental matters to understand the invention and then respective items.

[Description for Understanding Invention - 1]

(Single crystal)

The term "single crystal" refers to a crystal consisting of a single crystal grain such that the crystal axes, which specify the orientations of atomic arrangement, are uniform throughout the inside of the crystal grain. However, the single crystal of the present invention may include a lattice defect such as dislocation, a vacancy, a void, a heterogeneous phase, amorphous or an organic substance in the inside of the single crystal. In addition, the single crystal of the present invention may include a domain structure that is generated, for example, by difference in the crystal system or difference in the direction of spontaneous polarization due to phase transition in the inside.

(Perovskite type)

The term "perovskite type" refers to a perovskite structure that is ideally a cubic crystal structure as described in Iwanami Rikagaku Jiten 5th Ed. (Iwanami Shoten, Publishers, published on February 20, 1998). Perovskite-type oxides are generally represented as the chemical formula $ABO_3$. Although the mole ratio between the element at site A or site B and the element O is shown as 1:3, even an oxide in which oxygen vacancies are present in part of the crystal and an oxide such that the quantitative ratio of the elements is slightly deviated can be said to be a perovskite-type oxide as long as the oxide has a perovskite-type main phase. If the element A, the element B and the element O undergo slight coordinate shifts from the symmetrical positions in the unit cell, the perovskite-type unit cell distorts to result in a cubic, rhombohedral or orthorhombic crystal system.

The perovskite-type single crystal of the present invention contains a perovskite-type oxide, and the single crystal can take the form not only of a cubic crystal system but also of a tetragonal, rhombohedral or orthorhombic crystal system through one or more elements constituting A and B. Whether an oxide is of perovskite type or what crystal system the oxide has can be determined through structural analysis, for example, by X-ray diffraction or electron beam diffraction. In this case, a sample may be powdered before measurement, as necessary.

The following specifically describes the method of the present invention for manufacturing a perovskite-type single crystal.

(Producing first single crystal)

The first single crystal may be produced by any method of liquid-phase methods including the Czochralski method, the Bridgman method, a floating zone melting method and a flux method, solid-phase methods including a solid-phase reaction method and a sol-gel method, and gas-phase methods including a sublimation method and chemical vapor deposition, as long as the single crystal can be produced under an atmospheric environment.

The method of the present invention for manufacturing a single crystal includes step (1) of firing a raw material containing an acceptor under an atmospheric environment to produce a first single crystal of perovskite type.

(Acceptor)

The acceptor to be used in the method of the present invention for manufacturing a perovskite-type single crystal can be selected from the group consisting of elements with ions of which some ions of an element in a perovskite-type single crystal are substituted after undergoing reduction in valence.

Firing a raw material containing an acceptor under an atmospheric environment to obtain a first single crystal of perovskite type leads to an enhanced coercive field. When an ion of an element at a site of a single crystal of perovskite undergoes reduction in valence below the original valence and substituted with an acceptor, the charge balance of the crystal lattice is broken, and an oxygen vacancy is generated at an oxygen site to compensate the balance, resulting in the formation of a dipole of the acceptor and the oxygen vacancy to generate an internal electric field. The internal electric field hinders the inversion of polarization to be caused by an external electric field, leading to an enhanced coercive field.

As a result of firing a raw material containing an acceptor under an atmospheric environment, the acceptor is less likely to enter vacancies or the like as a substance other than the first single crystal, and substitution with the acceptor can be achieved at more sites of the perovskite-type crystal.

For example, in a perovskite-type oxide configured with $A^{2+}B^{4+}O_3$, one or two or more can be selected from the group consisting of Li, K, Na and so on as an acceptor for substitution at sites A. One or two or more can be selected from the group consisting of Mg, Mn, Zn, Fe, Co, Al, Ni, Cr, Y, Sc, In, Bi, Yb and so on as an acceptor for substitution at sites B.

In a perovskite-type oxide of $A^{1+}B^{5+}O_3$, one or two or more can be selected from the group consisting of Mg, Mn, Zn, Fe, Co, Ni, Cr, Y, Sc, In, Yb, Ti, Zr, Sn, Hf and so on as an acceptor for substitution at sites B.

In a perovskite-type oxide of $A^{3+}B^{3+}O_3$, one or two or more can be selected from the group consisting of Li, Na, K, Pb, Ba, Ca and so on as an acceptor for substitution at sites A. One or two or more can be selected from the group consisting of Mg, Mn, Fe, Co, Zn and so on as an acceptor for substitution at sites B.

Here, the raw material to be used as an acceptor may be metal, oxide, sulfide or nitride as long as the raw material contains any of those elements, and the state may be solid or liquid.

The acceptor to be used in the method of the present invention for manufacturing a single crystal can be Mn.

Mn covers a wide range of valence from divalent to heptavalent, and can serve as an acceptor for any of $A^{2+}B^{4+}O_3$, $A^{1+}B^{5+}O_3$ and $A^{3+}B^{3+}O_3$. If Mn is present as an acceptor in a perovskite-type single crystal, oxygen vacancies are formed in the single crystal, and a Mn ion and an oxygen vacancy forms a dipole to generate an internal electric field. The internal electric field hinders the inversion of polarization to be caused by an external electric field, leading to an enhanced coercive field.

In the case that conduction electrons are present because of generation of a donor element from an impurity or the like, Mn ions trap conduction electrons through undergoing reduction in valence, successfully leading to an enhanced insulation resistance, and thus Mn can be used.

The valence of Mn in a trace amount added into a non-magnetic (diamagnetic) material can be assessed by measurement of the temperature dependence of the magnetic susceptibility. Magnetic susceptibility can be measured by means of a superconducting quantum interference device (SQUID), a vibrating sample magnetometer (VSM) or a magnetic balance. Magnetic susceptibility $\chi$ to be obtained in the measurement generally obeys the Curie-Weiss law represented by the following expression 1:

$$\chi = C / (T - \theta) \quad (\text{C: Curie constant, } \theta\text{: paramagnetic Curie temperature})$$

(expression 1).

Mn in a trace amount added into a non-magnetic material generally exhibits spin S = 5/2 at a valence of 2+, s = 2 at 3+ and s = 3/2 at 4+. Accordingly, a Curie constant C converted into a value per unit amount of Mn is a value corresponding to values of spin S at different valences of Mn. Thus, the average valence of Mn in a sample can be assessed by deriving a Curie constant C from the temperature dependence of the magnetic susceptibility $\chi$.

(Firing under reducing environment and under atmospheric environment in this order)

The method of the present invention for manufacturing a perovskite-type single crystal is characterized by including step (2) including firing the first single crystal, which was produced with the above method, under a reducing environment and step (3) including firing the single crystal produced in step (2) under an atmospheric environment in this order. Through this method, a perovskite-type single crystal having a higher coercive field value than the first single crystal is produced.

The first single crystal of perovskite type that is produced by firing a raw material containing an acceptor under an atmospheric environment is inferred to contain substituting acceptor ions keeping the same valence as the original element and acceptor ions remaining in vacancy parts in a non-perovskite state. When such a first single crystal is fired under a reducing environment, oxygen vacancies are further generated to add to oxygen vacancies originally present in the first single crystal, and the residual acceptor ions as mentioned are inferred to be incorporated in sites in the single crystal.

As a result, more dipoles of an acceptor ion and an oxygen vacancy form to generate a more intense internal electric field. The internal electric field hinders the inversion of polarization to be caused by an external electric field, leading to a much enhanced coercive field.

Through firing under a reducing environment, the single crystal undergoes reduction in the maximum value of the relative dielectric constant $\varepsilon_{33}$ and reduction in the variation width (%) of the piezoelectric constant $d_{33}$.

In general, as the temperature of a piezoelectric material changes toward the phase transition temperature, the rearrangement or loss of polarization occurs, and the change of polarization to be caused by an external electric field, that is, the relative dielectric constant increases. This change is remarkable for single crystals. However, if the internal electric field is increased by an acceptor, the change of polarization to be caused by an external electric field is reduced to result in a lower relative dielectric constant. The piezoelectric constant $d_{33}$ is a function of the relative dielectric constant as shown by the following expression (2), and hence the variation width under temperature change is decreased for the same reason:

$$d_{33} = k\sqrt{\frac{\varepsilon^T}{Y}} \qquad (2)$$

wherein d denotes the piezoelectric constant, k denotes the electromechanical coupling factor, $\varepsilon^T$ denotes the dielectric constant as the product of the relative dielectric constant and the dielectric constant of vacuum, and Y denotes the Young's modulus.

(Firing under reducing environment)

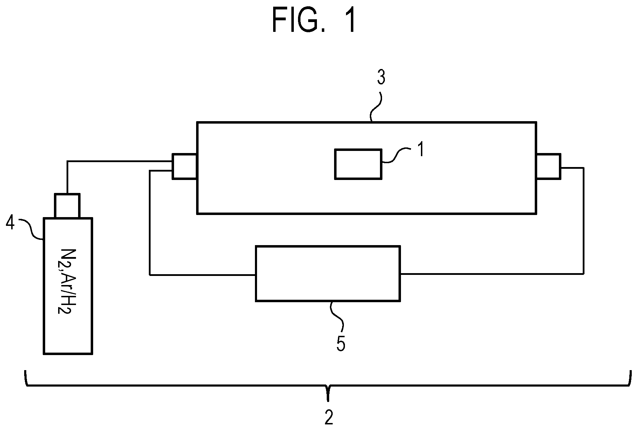

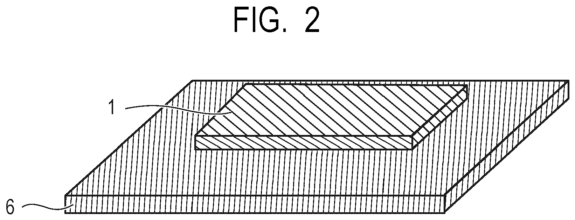

Next, step (2) including firing the first single crystal under a reducing environment will be specifically described with FIG. 1 and FIG. 2.

FIG. 1 is a schematic diagram illustrating the configuration of a reducing environment firing furnace 2 for firing a first single crystal 1 under a reducing environment. In FIG. 1, the first single crystal 1 is set in a high-temperature electric furnace 3, which is configured in such a manner that the atmosphere in the high-temperature electric furnace 3 can be purged with a gas such as an inert gas discharged from a gas cylinder 4. To control the oxygen partial pressure in the high-temperature electric furnace, an oxygen partial pressure controller 5 is set. For example, an SiOC-200CB (manufactured by STLab CO., LTD.) can be used as the oxygen partial pressure controller.

FIG. 2 shows an exemplary configuration diagram for setting the first single crystal 1 in the reducing environment firing furnace 2.

The first single crystal 1, which is placed on a setter 6, can be set in this state in the high-temperature electric furnace 3 in FIG. 1.

Materials quite unreactive with the first single crystal 1 are suitable for the setter 6; for example, alumina, zirconia, alumina coated with zirconia on the surface, stabilized zirconia, silicon carbide or silicon nitride can be used. If it is difficult to determine whether materials are quite unreactive or not, the materials can be actually tested to select a material with the least mark of firing.

(Reducing environment)

The reducing environment can be a space filled with an oxygen-free gas or vacuum, and one or more can be selected as the gas from the group consisting of hydrogen, helium, carbon monoxide, hydrocarbon, argon, nitrogen, ammonia and so on, and the gas can be an argon-containing gas. Among inert gasses, argon is quite unreactive with the first single crystal and hence allows more oxygen vacancies to be generated, resulting in a more enhanced coercive field. A reducing environment containing 90 mol% or more of argon can be used because the effect of generating more oxygen vacancies is remarkable.

Furthermore, the oxygen partial pressure P ($O_2$) of the reducing environment can be $1 \times 10^{-10}$ Pa or less. The oxygen partial pressure of $1 \times 10^{-10}$ Pa or less allows more oxygen vacancies to be generated, resulting in a more enhanced coercive field.

The firing temperature for firing under a reducing environment in step (2) can be equal to or lower than the firing temperature for producing the first single crystal in step (1), and can be 1280°C or more. Firing at a temperature lower than the temperature in producing the first single crystal is less likely to cause generation of a heterogenous phase, which is a crystal differing from perovskite, and the single crystal structure is likely to be maintained, and as a result a single crystal having a high electromechanical coupling factor is obtained.

(Firing under atmospheric environment)

Next, step (3) including firing the single crystal, which has been produced through step (2) including firing the first single crystal under a reducing environment, under an atmospheric environment will be described.

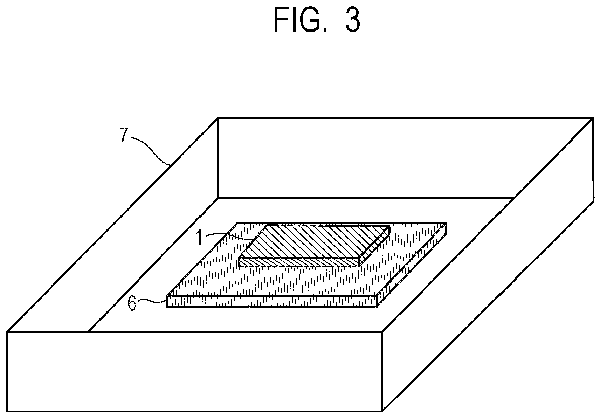

FIG. 3 is a schematic diagram for describing the first embodiment of the present invention.
Step (3) according to the present invention after firing the first single crystal 1 under a reducing environment includes firing the resultant under an atmospheric environment. As illustrated in FIG. 3, step (3) can be performed by placing the first single crystal 1, which has been fired in a reducing environment and set on a setter 6, in a saggar 7 and placing the saggar 7 in an electric furnace for firing. Firing may be performed with a lid (not shown) to prevent contamination with impurities from the surrounding.
Common electric furnaces can be used for firing under an atmospheric environment; for example, a super electric furnace (SSFT-1520 manufactured by YAMADA DENKI CO., LTD.) can be used.
The single crystal of the present invention produced by firing the first single crystal 1 under a reducing environment and then firing the resultant under an atmospheric environment exhibits a high electromechanical coupling factor when being used for a piezoelectric element because heterogenous phases generated in firing under a reducing environment have been removed.
The firing temperature for firing under an atmospheric environment in step (3) can be equal to or lower than the firing temperature for firing under a reducing environment in step (2), and can be 1000°C or less. Firing under an atmospheric environment at a temperature equal to or lower than the temperature in firing under a reducing environment allows oxygen vacancies introduced in firing under a reducing environment to be better maintained, resulting in a higher coercive field.

(Method for producing first single crystal)

Next, an example of specific production methods for producing a first single crystal 1 will be described.
Producing a first single crystal 1 in the present invention can be by a solid-phase growth method.
Producing a first single crystal 1 by a solid-phase growth method can be done because forming melt and cooling as in a liquid-phase method are not involved, and hence even in the case that an incongruent melting composition (incongruent composition) results by addition of an acceptor, a first single crystal 1 having almost the same composition as a raw material containing the acceptor can be produced.
The following describes an embodiment in which obtaining a first single crystal is by a solid-phase growth method.

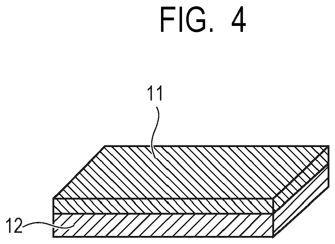

FIG. 4 is a schematic diagram illustrating an embodiment of an integrated product of a seed single crystal 11 and a matrix 12 in the present invention. In FIG. 4, the seed single crystal 11 is set on the top of the matrix 12 to form an integrated product in which a surface of the matrix 12 and a surface of the seed single crystal 11 are disposed in contact.
The method of the present invention for manufacturing a first single crystal is a manufacturing method of causing single crystal formation to the matrix 12 by firing the matrix 12 in the integrated product, for example, as illustrated in FIG. 4.

(Description of matrix)

The matrix 12 in FIG. 4 may be a molded body or a sintered body. The raw material that is used for the matrix 12 is solid powders of oxides, carbonates, nitrates, oxalates or the like of constituent elements of the single crystal of the present invention, including an acceptor, or a mixed powder of these solid powders.
Examples of elements constituting the raw material of the matrix 12 include Li, Na, Mg, Al, Si, K, Ca, Sc, Ti, V, Cr, Mn, Fe, Co, Ni, Cu, Zn, Ge, Sr, Y, Zr, Nb, Mo, In, Sn, Sb, Ba, Hf, Ta, Pb, Bi, La, Nd, Sm, Eu, Gd, Tb, Dy, Ho, Er and Yb.
Here, the case where the lead (Pb) content of the raw material is less than 1000 ppm is used because of low environmental loads.
In the case that the elements Ba, Ti and Zr are used and the element Mn is used as an acceptor, for example, examples of applicable Ba raw materials include barium oxide, barium carbonate, barium oxalate, barium acetate, barium nitrate, barium titanate and barium zirconate. For these Ba compounds, commercially available high-purity-type compounds (e.g., a purity of 99.99% or more) can be used.
Examples of applicable Ti raw materials include titanium oxide, barium titanate and barium titanate zirconate. In the case that an alkaline earth metal such as barium is contained in such a Ti compound, commercially available high-purity-type compounds (e.g., a purity of 99.99% or more) can be used.

Nb may be contained to the same degree as Nb is contained as an inevitable component in commercially available raw materials of Ti, and Hf may be contained to the same degree as Hf is contained as an inevitable component in commercially available raw materials of Zr.

Examples of applicable Zr raw materials include zirconium oxide, barium zirconate and barium titanate zirconate. In the case that alkaline earth metal such as barium is contained in such a Zr compound, commercially available high-purity-type compounds (e.g., a purity of 99.99% or more) can be used.

Examples of applicable Mn raw materials include manganese carbonate, manganese oxide, manganese dioxide, manganese acetate and trimanganese tetraoxide.

The raw material of the matrix 12 can contain Bi. If the Bi content is 0.20 parts by mass or less in terms of metal to 100 parts by mass of the perovskite-type metal oxide as a main component of the matrix 12, trivalent Bi ions can occupy sites A. This allows Mn ions at sites B to have a valence lower than 4+ for charge balance as a tendency, and facilitates Mn ions to serve as acceptor ions.

Examples of applicable Bi raw materials in the case that Bi is used for the raw material of the matrix 12 include bismuth oxide and bismuth chloride.

A compound that promotes single crystal formation by a solid-phase growth method may be added to the raw material of the matrix 12. Examples of promoter elements include lithium (Li), silicon (Si) and aluminum (Al), and the Li content can be 0.05 parts by mass or more and 0.20 parts by mass or less in terms of metal to 100 parts by mass of the perovskite-type metal oxide as a main component of the matrix 12.

Examples of applicable Li raw materials in the case that Li is used for the raw material of the matrix 12 include lithium carbonate, lithium tetraborate and lithium acetate.

Any raw material may be used without particular limitation to adjust a, which denotes the ratio between the abundance of Ba ions at sites A and the amount in moles of Ti and Zr at sites B in the single crystal according to the present invention. Ba compounds, Ti compounds and Zr compounds have the same effect.

(Granulation of raw material for producing matrix)

The raw material of the matrix 12 to be used in the method of the present invention for manufacturing a single crystal may have been granulated, and the granulation is not limited to any particular method. Examples of applicable binders in the granulation include PVA (polyvinyl alcohol), PVB (polyvinyl butyral) and acrylic resin.

(Molded body of raw material for producing matrix)

The molded body of the matrix 12 to be used in the method of the present invention for manufacturing a single crystal is a molded body produced by hardening the raw material into a desired shape, and the manufacture is not limited to a particular method. The molded body is a solid produced from a powder, granulated powder (with a binder) or slurry of the raw material.

For example, uniaxial pressing, cold hydrostatic pressing, warm hydrostatic pressing, cast molding or extrusion molding can be used as a means for producing the molded body. The raw material in the form of slurry may be shaped into a sheet by a doctor blade method and then dried, and such sheets may be stacked to form an intended shape.

(Sintering)

In the case that a sintered body is used as the matrix 12, the molded body can be sintered and the means for sintering is not limited, and examples of sintering methods include sintering with an electric furnace, sintering with a gas furnace, a hot-press method, an electric heating method, a microwave sintering method, a millimeter wave sintering method, HIP (hot isostatic press) and a flash sintering method.

(Seed single crystal)

The seed single crystal 11 for use in producing the first single crystal may be a bulk or a thin film. The seed single crystal 11 can be prepared by selecting from the group consisting of commercially available oxide singe crystals, semiconductor single crystals, fluoride-alkali halide single crystals, metal single crystals, alloy single crystals and so on, but perovskite-type single crystals can be used because of the low tendency to generate stress due to the mismatch in lattice constants and difference in thermal expansion for the matrix 12.

Examples of perovskite-type single crystals for use as the seed single crystal 11 include $BaTiO_3$, $BaZrO_3$, $SrTiO_3$, $CaTiO_3$, $Pb(Mg_{1/3}Nb_{2/3})O_3$, $KTaO_3$, $KNbO_3$, $SrRuO_3$, $LiNbO_3$, $LaNiO_3$, $AgNbO_3$, $CdTiO_3$ and a solid solution of any of these perovskite-type single crystals.

A first single crystal produced through the above-described method may be used as the seed single crystal 11 for use in producing a first single crystal.

(Polishing)

The matrix 12 obtained by sintering as described above and the seed single crystal 11 are polished, and the polished surfaces of the matrix 12 and seed single crystal 11 are disposed in contact to form an integrated product; as a result, a larger contact area can be achieved.

Polishing methods include mechanical polishing, chemical polishing and CMP polishing, and polishing can be performed with a combination of these polishing methods.

(Saggar, setter, beads, weight)

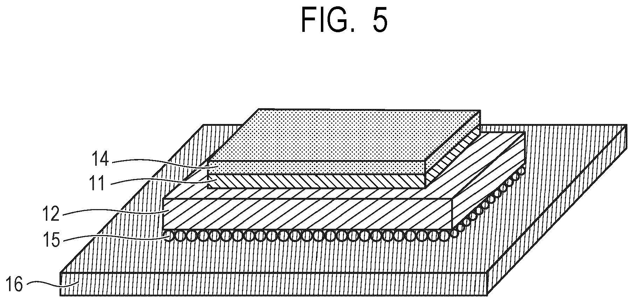

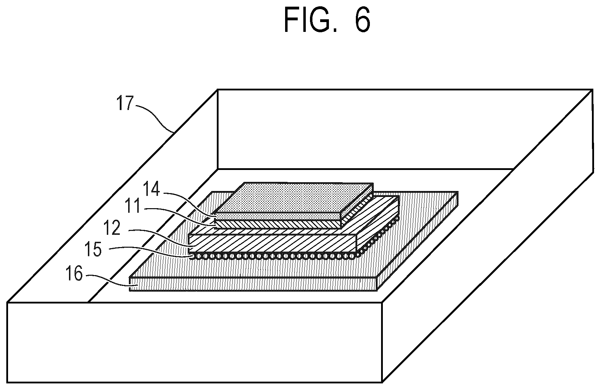

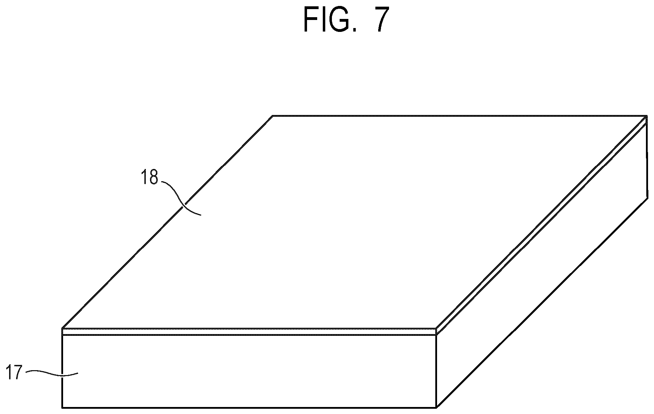

FIG. 5, FIG. 6 and FIG. 7 are exemplary schematic diagrams illustrating the configuration to produce a first single crystal. In FIG. 5, a weight 14 lies on the top of the integrated product of the matrix 12 and the seed single crystal 11, and the integrated product is set above a setter 16 via beads 15. FIG. 6 shows the situation in which they are placed in a saggar 17, and FIG. 7 shows the situation in which the saggar 17 in FIG. 6 is covered with a lid 18, and the saggar 17 covered with the lid 18 is placed in an electric furnace for firing.

The matrix 12 and the seed single crystal 11 can be disposed vertically to come into contact. A weight 14 can be laid on the top of the matrix 12 and seed single crystal 11 because the matrix 12 and the seed single crystal 11 can come into contact with ease. Even if force acts in the direction to separate the matrix 12 and the seed single crystal 11 from each other because of deformation or the like in heat treatment, the weight 14 applies force that acts in the direction to prevent the separation, facilitating the contact.

The matrix 12 and the seed single crystal 11 can be subjected to heat treatment on a setter 16 quite unreactive with the matrix 12 and the seed single crystal 11. For the material of the setter 16, for example, alumina, zirconia, alumina coated with zirconia on the surface, stabilized zirconia, silicon carbide or silicon nitride can be used. The matrix 12 and seed single crystal 11 may be placed on the top of beads 15 of stabilized zirconia or the like, a powder obtained by sintering the matrix 12 at high temperature and crushing the resultant, or a platinum sheet that has been set on the setter 16. If it is difficult to determine whether materials are quite unreactive or not, the materials can be actually tested to select a material with the least mark of firing.

(Liquid phase of matrix)

The matrix 12 for use in producing the first single crystal 1 has a liquid-phase-appearing temperature and a liquid-phase-disappearing temperature. The term "liquid phase" here refers to a state in which at least part of the matrix 12 is in a liquid phase, and a state in which a solid phase and a liquid phase coexist is also regarded as a liquid phase. For example, the state and temperature can be confirmed in such a manner that a sample is placed on a heating stage for microscopes (manufactured by Linkam Scientific Instruments Ltd.) and the temperature is increased, and the surface of the matrix 12 is microscopically observed. Specifically, a temperature at which at least part of grain boundaries of the matrix 12 has become unobservable, which indicates transition into a liquid phase, during temperature increase is the liquid-phase-appearing temperature, and a temperature at which grain boundaries of the matrix 12 are at least partly generated through further temperature increase is the liquid-phase-disappearing temperature.

Because a liquid phase becomes amorphous through rapid cooling, as another means, the temperature at transition into a liquid phase can be confirmed through structural analysis by X-ray diffraction on the presence or absence of an amorphous region for the matrix 12 that has been subjected to temperature increase to heat close to the liquid-phase temperature and then directly put in tap water or the like at room temperature for rapid cooling.

The liquid-phase-appearing temperature in the present invention varies by the influence of contamination with impurities in preparing, pressure unevenness in molding, voids and so on. Accordingly, a range within ±50°C from the lowest temperature at which a liquid phase can be confirmed by the above means is defined as the liquid-phase-appearing temperature. A range within ±50°C from the highest temperature at which a liquid phase can be confirmed by the above means is defined as the liquid-phase-disappearing temperature.

When an interface between the seed single crystal 11 and the matrix 12 transforms into a liquid phase, the interface of the single crystal region of the seed single crystal 11 gradually propagates to the matrix 12. At that time, crystal grains of the matrix 12 are inferred to be incorporated into the single crystal because of the difference in surface energy. Therefore, excessively large crystal grains of the matrix 12 result in smaller difference in surface energy, and as a result the matrix 12 is less likely to be incorporated into the single crystal.

Thus, in the case that the matrix 12 is prepared as a sintered body, the matrix 12 has been sintered at a temperature that does not cause the formation of excessively large crystal grains. As a means for preventing crystal grains from increasing in size, a method of sintering under a reducing environment such as nitrogen and argon as a sintering reducing environment may be used.

(Firing method)

In first temperature increase, second temperature increase and firing described later, firing the integrated product of the matrix 12 and the seed single crystal 11 is not limited to a particular method as long as firing is performed under an atmospheric environment, but firing with an electric furnace can be done in that many integrated products can be fired at once and reduction in production cost is achieved.

[First temperature increase]

The method for producing a first single crystal 1 includes first temperature increase of increasing the temperature of the integrated product from room temperature to the liquid-phase-appearing temperature of the matrix.
In the first temperature increase of increasing the temperature to the liquid-phase-appearing temperature, almost homogeneous crystal grains grow in the matrix. As described above, if crystal grains of the matrix 12 grow excessively large, the matrix 12 is less likely to be incorporated into the first single crystal, and hence the first temperature increase can be set to be performed for a short time so as not to allow crystal grains to grow excessively large.

[Second temperature increase]

The method for producing a first single crystal 1 includes second temperature increase of increasing the temperature of the integrated product from the liquid-phase-appearing temperature to the liquid-phase-disappearing temperature of the matrix 12.
In the second temperature increase, an interface between the seed single crystal 11 and the matrix 12 transforms into a liquid phase, and the single crystal interface propagates to the matrix 12. However, if the temperature has spatial variation, for example, due to difference in orientation of crystal grains of the matrix 12 in contact with the seed single crystal 11, the interface of crystal grains with different orientation in the matrix 12 also simultaneously propagates, and a single crystal as a single crystal grain is not successfully produced in some cases.
Accordingly, by employing a temperature increase rate from the liquid-phase-appearing temperature to the liquid-phase-disappearing temperature in the second temperature increase smaller than the temperature increase rate from room temperature to the liquid-phase-appearing temperature in the first temperature increase allows the single crystal region consisting of a single crystal grain to propagate from the seed single crystal 11.
After the temperature increase, the single crystal region that has propagated from the seed single crystal 11 can be further increased in size by retaining the temperature at the liquid-phase-disappearing temperature.

[Producing first single crystal]

The method for producing a first single crystal 1 includes producing a first single crystal 1 from the matrix 12 after being fired.
Where the single crystal region is in the matrix after the second temperature increase can be determined by checking the presence or absence of a grain boundary with a microscope. A region without any grain boundary is the single crystal region consisting of a single crystal grain. Alternatively, the single crystal region can be confirmed through structural analysis by X-ray diffraction or through electron beam diffraction.
The single crystal region can be isolated, thus yielding a first single crystal, by removing polycrystalline parts other than the single crystal region and the seed single crystal 11 part with a wire saw or a dicing saw, or by means of any of water-jet cutting, electric discharge machining, laser machining, polishing and so on.
The first single crystal is a single crystal containing a perovskite-type oxide containing Ba, Ti and Zr, and Mn, with x as the mole ratio of Zr to the sum total of Ti and Zr satisfying $0.02 \leq x \leq 0.13$, and has a Mn content of 0.04 parts by mass or more and 0.36 parts by mass or less in terms of metal to 100 parts by mass of the oxide.
The inclusion of Zr and Mn in amounts satisfying those ranges results in less occurrence of abnormal grain growth in parts other than the interface between the seed single crystal 11 and the matrix 12. If abnormal grain growth occurs in a part other than the interface between the seed single crystal 11 and the matrix 12, the part hinders the growth of the single crystal propagating from the interface between the seed single crystal 11 and the matrix 12. The first single crystal can contain an oxide containing Ba, Ti and Zr, and Mn, Bi and Li, with x as the mole ratio of Zr to the sum total of Ti and Zr satisfying $0.02 \leq x \leq 0.13$, and has a Mn content of 0.04 parts by mass or more and 0.36 parts by mass or less in terms of metal to 100 parts by mass of the oxide, a Bi content of more than 0 parts by mass and 0.20 parts by mass or less in terms of metal to 100 parts by mass of the oxide, and a Li content of 0.05 parts by mass or more and 0.20 parts by mass or less in terms of metal to 100 parts by mass of the oxide.

Through step (2) including firing the first single crystal produced by the solid-phase growth method under a reducing environment and step (3) including firing the single crystal produced in step (2) under an atmospheric environment in order, the perovskite-type single crystal of the present invention, which has a higher coercive field value than the first single crystal, is produced.

<Second Embodiment>

The second embodiment relates to the perovskite-type single crystal of the present invention.

The perovskite-type single crystal of the present invention is a single crystal containing an oxide containing Ba, Ti and Zr with perovskite structure, and Mn, with x as the mole ratio of Zr to the sum total of Ti and Zr satisfying $0.02 \leq x \leq 0.13$, wherein the single crystal has a Mn content of 0.04 parts by mass or more and 0.36 parts by mass or less in terms of metal to 100 parts by mass of the oxide, the single crystal optionally contains Bi, and has a Bi content of 0 parts by mass or more and 0.20 parts by mass or less in terms of metal to 100 parts by mass of the oxide, and the single crystal has an electromechanical coupling factor $k_{33}$ of 80% or more at 25°C and a coercive field of 2.5 kV/cm or more.

Here, the Mn content "in terms of metal" indicates a quantity given as follows: the metal contents of the single crystal are measured for Ba, Ti, Zr and Mn by X-ray fluorescence analysis (XRF), ICP emission spectrometry, atomic absorption spectrometry or the like; the metal contents are converted into the masses of oxides of elements constituting an oxide represented by the general formula (3) shown below; the ratio of the mass of Mn to the total mass as 100 is determined, and the resulting value is the Mn content.

$$Ba_a(Ti_{1-x},Zr_x)O_3 \qquad (3)$$

The single crystal of the present invention can contain less than 1000 ppm of a Pb component. Being free of lead, the single crystal of the present invention can achieve low environmental loads.

The general formula (3) for an oxide means that the element positioned at sites A is Ba and the element positioned at sites B is Ti or Zr. However, some Ba ions may be positioned at sites B. Similarly, some Ti and Zr ions may be positioned at sites A.

While the mole ratio between the elements at sites B and the element O in the general formula (3) is 1:3, the ratio of the element contents may be deviated, for example, by oxygen vacancies generated through substitution with Mn at sites B.

The single crystal of the present invention can contain 90 mol% or more of the perovskite-type oxide represented by the general formula (3) as a main component. A further range is 95 mol% or more.

In the general formula (3), x, which denotes the mole ratio of Zr at sites B, is in the range of $0.02 \leq x \leq 0.13$. If x is more than 0.13, the Curie temperature becomes excessively low, resulting in insufficient high-temperature durability. If x is less than 0.02, a low piezoelectric constant results at room temperature.

Curie temperature (Tc) is a temperature at which the ferroelectricity of a material disappears. Typically, the piezoelectric performance of a piezoelectric material disappears when the temperature has increased to exceed Tc. Methods for measuring Tc include a method of directly determining the temperature at which ferroelectricity disappears while the measurement temperature is varied, and a method in which the relative dielectric constant is measured while the measurement temperature is varied with a minute alternating electric field and Tc is determined from the temperature at the local maximum of the relative dielectric constant.

Measurement of the composition of the single crystal of the present invention is not limited to a particular means. Examples of the means include X-ray fluorescence analysis, ICP emission spectrometry and atomic absorption spectrometry. The mass ratio and composition ratio of the elements contained in the single crystal can be calculated with any of those means.

Containing Mn in an amount satisfying the above range, the single crystal of the present invention has an enhanced insulation resistance. In using a piezoelectric element including the single crystal and a pair of electrodes as a device, the high insulation resistance resulting from the Mn content being 0.04 parts by mass or more and 0.36 parts by mass or less does not cause an unnecessary current to flow when the device is driven by applying a voltage, and hence low electric power consumption is achieved.

If the Mn content is more than 0.36 parts by mass, on the other hand, the abundance of heterogenous phases differing from the perovskite-type single crystal is higher, and the single crystal has lower insulation properties. For example, the dielectric loss tangent of the single crystal at a frequency of 1 kHz may exceed 0.01, and the resistivity may fall below 1 GΩcm. The dielectric loss tangent can be measured with an impedance analyzer. If the dielectric loss tangent is 0.01 or less, stable operation is successfully achieved even when a high voltage is applied to a piezoelectric element for which the single crystal is used.

The single crystal can polarize if the single crystal has a resistivity of at least 1 GΩcm, and can be driven as a piezoelectric element. Additionally, the resistivity is 50 GΩcm or more.

Having an electromechanical coupling factor $k_{33}$ of 80% or more at 25°C, the single crystal, in using a piezoelectric element including the single crystal and a pair of electrodes as a vibrating device, achieves high efficiency of conversion from electric energy to mechanical energy, and low electric power consumption is achieved.

Having a coercive field of 2.5 kV/cm or more, the single crystal, in using a piezoelectric element including the single crystal and a pair of electrodes as a vibrating device, allows the piezoelectric element to be driven without loss of piezoelectric performance, even in a product to be driven at high voltages such as an ultrasonic motor and a piezoelectric acoustic part.

The single crystal can have a coercive field of 4 kV/cm or more in use for a vibrating device. This range allows a wider variety of products to be driven without loss of piezoelectric performance.

The variation width (%) of the piezoelectric constant $d_{33}$ of the single crystal of the present invention can be 65% or less, the variation width being represented by the following expression (4):

$$100 \times \Delta d_{33@0°C \text{ to } 70°C} / d_{33@0°C} \quad (4).$$

The maximum value of the relative dielectric constant $\varepsilon_{33}$ of the single crystal of the present invention in the range of 0°C or more and 70°C or less can be 6500 or less.

The maximum value of the relative dielectric constant $\varepsilon_{33}$ of the single crystal of the present invention in the range of 0°C or more and 60°C or less can be 4200 or less.

As described above, a piezoelectric material formed as a single crystal generally undergoes very large variation of the relative dielectric constant $\varepsilon_{33}$ and piezoelectric constant $d_{33}$ around the phase transition temperature. Accordingly, when a single crystal having piezoelectricity is used for a device in a product like a home appliance, such as an ultrasonic motor in a camera, increase in the relative dielectric constant of the piezoelectric material or variation in the piezoelectric constant (e.g., variation over 65%) may occur in the operation temperature range for the product (e.g., 0°C or more and 70°C or less). If the relative dielectric constant has increased, the capacitance component of the drive circuit increases, and the current flowing in the circuit increases. This disadvantageously results in larger electric power consumption in driving. In addition, the variation in the piezoelectric constant disadvantageously alters the output characteristics of the device (mainly, amplitude of vibration), resulting in poor controllability.

Therefore, in using a single crystal for a product like a home appliance, the single crystal is required to undergo small variation in the piezoelectric constant in the operation temperature range for the product. With the variation width of the piezoelectric constant on the influence of temperature being 65% or less, stable product performance is obtained in the operation temperature range.

With the maximum value of the relative dielectric constant $\varepsilon_{33}$ in the range of 0°C or more and 70°C or less being 6500 or less, lower electric power consumption is achieved in use in the operation temperature range.

With the maximum value of the relative dielectric constant $\varepsilon_{33}$ in the range of 0°C or more and 60°C or less being 4200 or less, which is a temperature range that is more frequently used, much lower electric power consumption is achieved in the operation temperature range.

<Application Examples>

The following describes application examples of the present invention.

(Piezoelectric element)

The piezoelectric element of the present invention includes a plurality of electrodes and the single crystal described above.

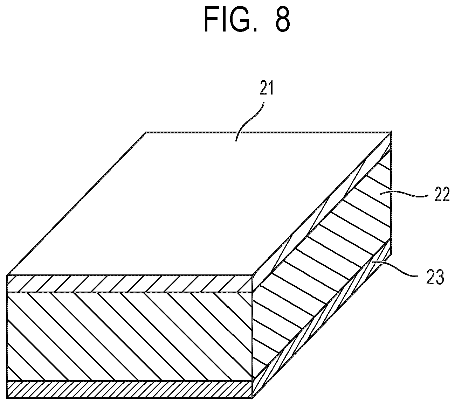

FIG. 8 is a schematic diagram illustrating an embodiment of the configuration of the piezoelectric element of the present invention. The piezoelectric element according to the present invention is a piezoelectric element at least including a first electrode 21, a single crystal part 22 and a second electrode 23, and characterized in that the single crystal part 22 is the single crystal of the present invention.

The electromechanical coupling factor, coercive field, piezoelectric constant and relative dielectric constant of the single crystal according to the present invention can be assessed with the single crystal incorporated in a piezoelectric element at least including a first electrode 21 and a second electrode 23. The first electrode 21 and second electrode 23 each include a conductive layer having a thickness of about 5 nm to 10 $\mu$m. The material is not limited to a particular material, and can be a material commonly used for piezoelectric elements. Examples the material can include metals such as Ti, Pt, Ta, Ir, Sr, In, Sn, Au, Al, Fe, Cr, Ni, Pd, Ag and Cu, and alloys and compounds of any of these metals.

The first electrode 21 and second electrode 23 may be each an electrode consisting of one of those materials, or an electrode obtained by laminating two or more of those materials. The material of the first electrode 21 and the material of the second electrode 23 may be different from each other.

Manufacture of the first electrode 21 and second electrode 23 is not limited to a particular method, and the first electrode 21 and second electrode 23 may be formed by baking with metal paste, or by sputtering, a vapor deposition method or the like. Each of the first electrode 21 and the second electrode 23 may be formed by patterning to produce a desired shape for use.

(Polarization treatment)

The polarization axes in the piezoelectric element can be aligned in a certain direction. The alignment of the polarization axes in a certain direction imparts a higher electromechanical coupling factor to the piezoelectric element.

Polarization of the piezoelectric element is not limited to a particular method. The polarization treatment may be performed in the atmosphere or in silicone oil. The temperature in polarization can be the temperature at which the single crystal undergoes phase transition. For example, the temperature can be 60°C to 150°C, but the optimum conditions depend to some extent on the composition of the single crystal constituting the element. The intensity of the electric field to be applied for polarization treatment can be 8 kV/cm to 20 kV/cm, and completing application of an electric field after the environmental temperature is lowered to a temperature at which the single crystal comes to take the same crystal structure as at room temperature is done because a good electromechanical coupling factor is obtained.

The scope of the piezoelectric element according to the present invention includes a laminate piezoelectric element obtained by laminating many layers each being the single crystal of the present invention or obtained by stacking many piezoelectric elements into a rod for making use of displacement in the thickness direction.

(Ultrasonic motor)

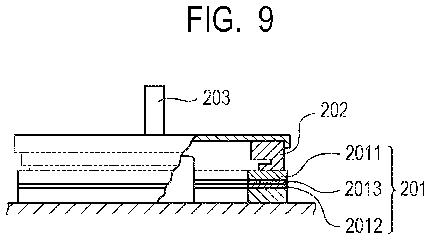

The ultrasonic motor of the present invention includes: a vibration unit provided with the piezoelectric element described above; and a mobile unit in contact with the vibration unit. By virtue of this configuration, an ultrasonic motor having a drive efficiency comparable to or higher than the drive efficiency in the case that a lead-containing piezoelectric element is used can be provided. FIG. 9 is a schematic diagram illustrating a mode of the configuration of the ultrasonic motor of the present invention. An ultrasonic motor in which the piezoelectric element of the present invention includes a single plate is illustrated in FIG. 9. The ultrasonic motor includes: a vibrator 201; a rotor 202 in contact with the sliding surface of the vibrator 201 by the action of pressurizing force applied by a pressurizing spring, which is not shown; and an output shaft 203 integrally provided on the rotor 202. The vibrator 201 is configured with an elastic ring 2011 made of metal, the piezoelectric element 2012 of the present invention and an organic adhesive 2013 (epoxy, cyanoacrylate or the like) adhering the piezoelectric element 2012 to the elastic ring 2011. The piezoelectric element 2012 of the present invention is configured with a single crystal sandwiched between a first electrode 21 and a second electrode 33, which are not shown. When two-phase alternating voltages with two phases offset from each other by an odd multiple of $\pi/2$ are applied to the piezoelectric element of the present invention, a flexural progressive wave is generated in the vibrator 201, and each point on the sliding surface of the vibrator 201 elliptically moves. If the rotor 202 is in pressure-contact with the sliding surface of the vibrator 201, the rotor 202 experiences frictional force from the vibrator 201, and rotates to the direction reverse to the direction of the flexural progressive wave. A driven unit, which is not shown, is jointed to the output shaft 203, and driven by the rotational force of the rotor 202. When a voltage is applied to the single crystal, the single crystal undergoes stretching vibration by the piezoelectric transverse effect. The efficiency of this conversion from electric energy to mechanical energy depends on the size of the electromechanical coupling factor. If an elastic body such as metal is adhered to a piezoelectric element via an adhesive or the like, the elastic body is bent via the adhesive by the stretching vibration of the single crystal. The ultrasonic motor of the type described here takes advantage of this principle.

(Optical device)

The optical device of the present invention includes the ultrasonic motor described above in a drive section. By virtue of this configuration, an optical device having an operation speed and operation efficiency comparable to or higher than the operation speed and operation efficiency in the case that a lead-containing piezoelectric element is used can be provided.

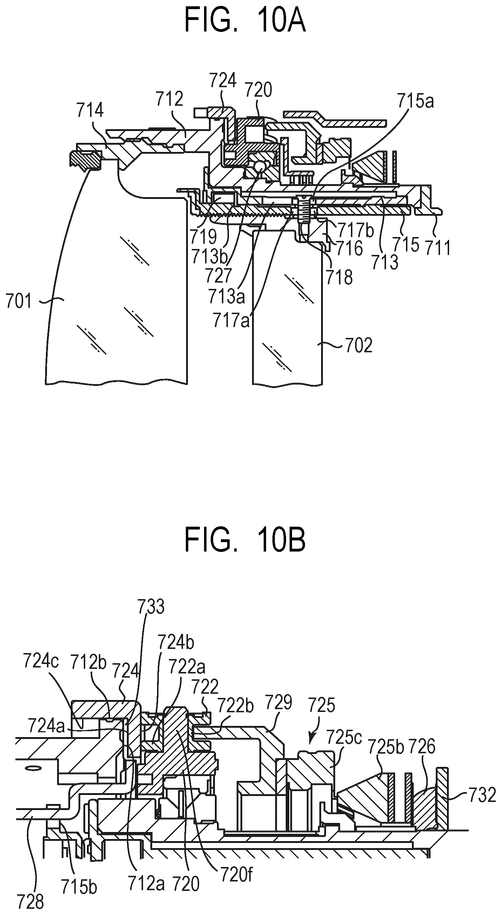

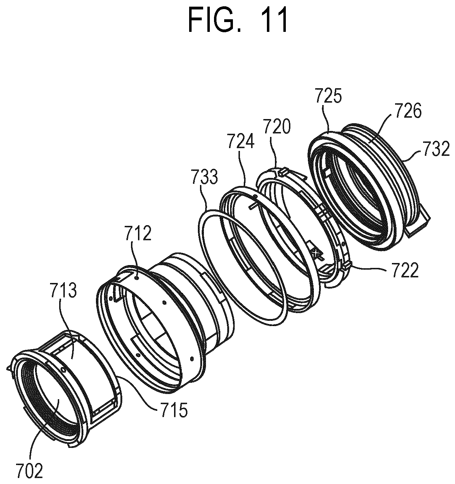

FIG. 10A and FIG. 10B are main cross-sectional views of an interchangeable lens barrel of a single-lens reflex camera as an example of embodiments of the optical device of the present invention. FIG. 11 is an exploded perspective view of an interchangeable lens barrel of a single-lens reflex camera as an example of embodiments of the optical device of the present invention. To a mount 711 for attachment and detachment of the camera, a fixing barrel 712, a straight guide barrel 713 and a front lens barrel 714 are fixed. These are fixed members of the interchangeable lens barrel.

In the straight guide barrel 713, a straight guide groove 713a for a focus lens 702 is formed in the optical axis direction. To a rear lens barrel 716 holding the focus lens 702, cam rollers 717a and 717b protruding outward in the radial direction are fixed with an axial screw 718, and the cam roller 717a fits in the straight guide groove 713a.

A cam ring 715 is rotatably fits in the inner periphery of the straight guide barrel 713. The relative movement of the straight guide barrel 713 and the cam ring 715 to the optical axis direction is restricted by the configuration in which a roller 719 fixed to the cam ring 715 fits in a peripheral groove 713b of the straight guide barrel 713. A cam groove 715a for the focus lens 702 is formed in the cam ring 715, and the aforementioned cam roller 717b simultaneously fits in the cam groove 715a.

On the outer periphery side of the fixing barrel 712, a rotation transmission ring 720 held by a ball race 727 pivotably to the fixing barrel 712 is disposed. In the rotation transmission ring 720, a runner 722 is rotatably held to a shaft 720f radially extending from the rotation transmission ring 720, and a large-diameter part 722a of the runner 722 is in contact with the mount-side end face 724b of a manual focus ring 724. A small-diameter part 722b of the runner 722 is in contact with a joint member 729. Six runners 722 are disposed on the outer periphery of the rotation transmission ring 720 at equal intervals, and each runner is configured to satisfy the above relationship.

A low-friction sheet (washer member) 733 is disposed on the inner peripheral part of the manual focus ring 724, and the low-friction sheet is sandwiched between the mount-side end face 712a of the fixing barrel 712 and the front-side end face 724a of the manual focus ring 724. The outer peripheral face of the low-friction sheet 733 is ring-shaped and peripherally fits to the inner periphery 724c of the manual focus ring 724, and the inner periphery 724c of the manual focus ring 724 peripherally fits to the outer peripheral part 712b of the fixing barrel 712. The low-friction sheet 733 plays a role in reducing friction in the rotational ring mechanism with the configuration in which the manual focus ring 724 rotates around the optical axis relatively to the fixing barrel 712.

The large-diameter part 722a of each runner 722 and the mount-side end face 724b of the manual focus ring are brought into contact with each other by pressurizing force applied through the force given by a wave washer 726 to press an ultrasonic motor 725 toward the front side of the lens. Likewise, the small-diameter part 722b of each runner 722 and the joint member 729 are brought into contact with each other by moderate pressurizing force applied through the force given by the wave washer 726 to press the ultrasonic motor 725 toward the front side of the lens. The movement of the wave washer 726 in the mount direction is regulated by a washer 732 bayonet-connected to the fixing barrel 712, and spring force (urging force) generated by the wave washer 726 is transmitted to the ultrasonic motor 725 and further to the runners 722, and also serves as force for the manual focus ring 724 to press the mount-side end face 712a of the fixing barrel 712. That is, the manual focus ring 724 is incorporated in such a manner that the manual focus ring 724 is pressed onto the mount-side end face 712a of the fixing barrel 712 via the low-friction sheet 733.

Accordingly, when the ultrasonic motor 725 is rotationally driven against the fixing barrel 712 by a control section, which is not shown, each runner 722 rotates around the center of the corresponding shaft 720f because the joint members 729 are in friction-contact with the small-diameter parts 722b of the runners 722. The rotation of each runner 722 around the corresponding shaft 720f results in the rotation of the rotation transmission ring 720 around the optical axis (autofocus operation).

When rotational force around the optical axis is given to the manual focus ring 724 from a manual operation input section, which is not shown, each runner 722 rotates around the corresponding shaft 720f by the action of frictional force because the mount-side end face 724b of the manual focus ring 724 is brought into pressure contact with the large-diameter part 722a of each runner 722. When the large-diameter part 722a of each runner 722 rotates around the corresponding shaft 720f, the rotation transmission ring 720 rotates around the optical axis. At that time, frictional retention force given by a rotor 725c and a stator 725b does not allow the ultrasonic motor 725 to rotate (manual focus operation).

Two focus keys 728 are attached to the rotation transmission ring 720 at positions opposite to each other, and each focus key 728 fits in a notched section 715b provided at the end of the cam ring 715. Accordingly, when the rotation transmission ring 720 is rotated around the optical axis through autofocus operation or manual focus operation, the rotational force is transmitted to the cam ring 715 via the focus keys 728. When the cam ring is rotated around the optical axis, the rear lens barrel 716, the rotation of which is restricted by the cam roller 717a and the straight guide groove 713a, is allowed to go forward or back along the cam groove 715a of the cam ring 715 by the cam roller 717b. This drives the focus lens 702, and thus focus operation works.

While an interchangeable lens barrel of a single-lens reflex camera has been described as the optical device of the present invention, the optical device of the present invention can be applied to any optical device including an ultrasonic motor in the drive section in all kinds of cameras such as a compact camera, an electronic still camera and a personal digital assistant with a camera.

(Vibration device and dust removal device)

The vibration device of the present invention includes a vibration unit with a diaphragm provided with the piezoelectric element described above. By virtue of this configuration, a vibration device having a vibration ability comparable to or higher than the vibration ability in the case that a lead-containing piezoelectric element is used can be provided.

Vibration devices, for example, for use in conveying or removing particles, powder or droplets are widely used in electronic devices and the like.

The following describes a dust removal device with the vibration device of the present invention as an example of the vibration device of the present invention.

The dust removal device according to the present invention includes a vibration device in a vibration section. By virtue of this configuration, a dust removal device having a dust removal efficiency comparable to or higher than the dust removal efficiency in the case that a lead-containing piezoelectric element is used can be provided.

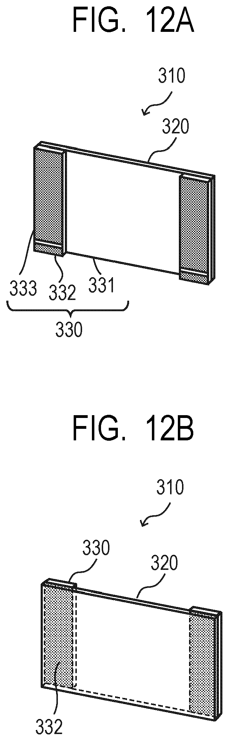

FIG. 12A and FIG. 12B are schematic diagrams illustrating a mode of the dust removal device of the present invention. A dust removal device 310 is configured with plate-like piezoelectric elements 330 and a diaphragm 320. The material of the diaphragm 320 is not limited, and in the case that the dust removal device 310 is used for an optical device, a translucent material or a light-reflective material can be used for the diaphragm 320.

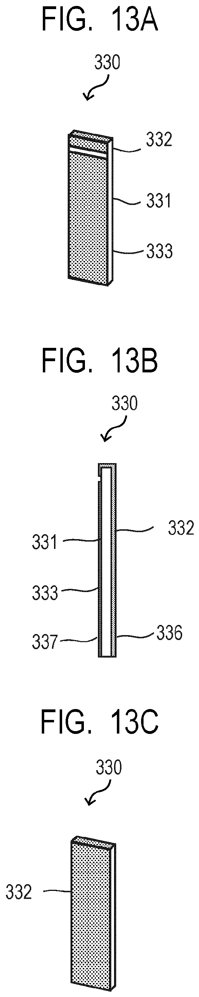

FIG. 13A to FIG. 13C are schematic diagrams illustrating the configuration of each piezoelectric element 330 in FIG. 12A and FIG. 12B. FIG. 13A and FIG. 13C respectively illustrate the configurations of the front and back faces of each piezoelectric element 330, and FIG. 13B illustrates the configuration of a side face of each piezoelectric element 330. As illustrated in FIG. 12A, each piezoelectric element 330 is configured with a single crystal 331, a first electrode 332 and a second electrode 333, and the first electrode 332 and the second electrode 333 are disposed to face the plate faces of the single crystal 331.

In FIG. 13B, the face on which the first electrode 332 lying on the front side of the piezoelectric element 330 is set is defined as a first electrode face 336; in FIG. 13A, the face on which the second electrode 333 lying on the front side of the piezoelectric element 330 is set is defined as a second electrode face 337.

Here, the term "electrode face" in the present invention refers to a face of the piezoelectric element on which an electrode is set; for example, as illustrated in FIG. 13A and FIG. 13B, the first electrode 332 may come around to the second electrode face 337.

As illustrated in FIG. 12A and FIG. 12B, the piezoelectric elements 330 and the diaphragm 320 are firmly fixed together with the first electrode face 336 of each piezoelectric element 330 set on a plate face of the diaphragm 320. Stress is generated between the piezoelectric elements 330 and the diaphragm 320 through the drive of the piezoelectric elements 330, generating an out-of-plane vibration of the diaphragm. The dust removal device 310 of the present invention is a device that removes foreign substances such as dust attached to the surface of the diaphragm 320 through the out-of-plane vibration of the diaphragm 320. The term "out-of-plane vibration" refers to an elastic vibration causing the diaphragm to displace in the optical axis direction, that is, the thickness direction of the diaphragm.

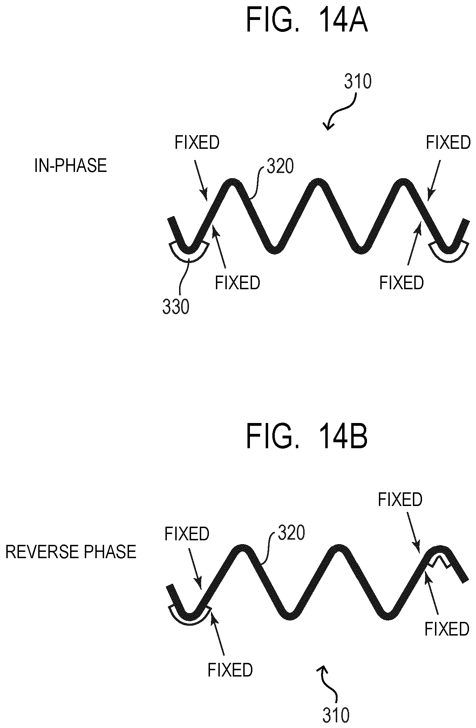

FIG. 14A and FIG. 14B are schematic diagrams illustrating the principle of the vibration of the dust removal device 310 of the present invention. FIG. 14A illustrates a situation in which the diaphragm 320 is caused to generate an out-of-plane vibration by applying in-phase alternating voltages to a pair of right and left piezoelectric elements 330. The direction of polarization of the single crystal constituting each of the pair of right and left piezoelectric elements 330 is the same as the thickness direction of the piezoelectric element 330, and the dust removal device 310 is driven in a seventh vibration mode. FIG. 14B illustrates a situation in which the diaphragm 320 is caused to generate an out-of-plane vibration by applying reverse-phase alternating voltages, the phases of which are 180°-reverse to each other, to a pair of right and left piezoelectric elements 330. The dust removal device 310 is driven in a sixth vibration mode. The dust removal device 310 of the present invention is a device that can effectively remove dust attached to the surface of the diaphragm by using at least two different vibration modes.

(Imaging device)

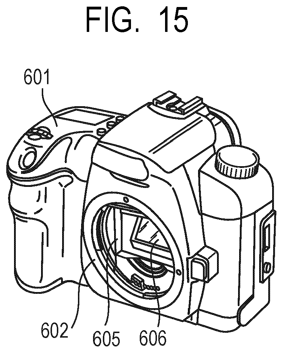

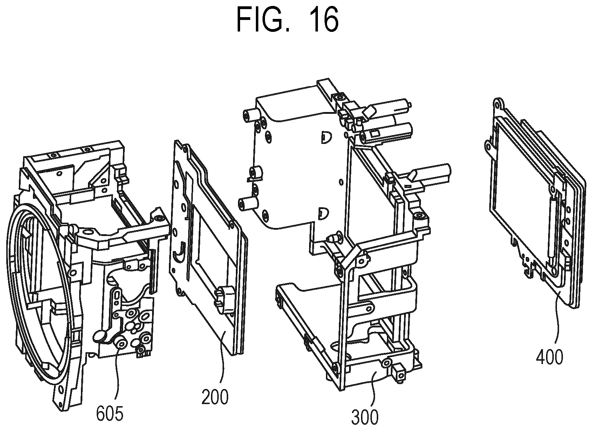

The imaging device of the present invention is an imaging device at least including the dust removal device described above and an imaging element unit, wherein the diaphragm of the dust removal device is provided on the light-receiving-face side of the imaging element unit. By virtue of this configuration, an imaging device having a dust removal function comparable to or higher than the dust removal function in the case that a lead-containing piezoelectric element is used can be provided. FIG. 15 and FIG. 16 are diagrams illustrating a digital single-lens reflex camera as an example of embodiments of the imaging device of the present invention.

FIG. 15 is a front perspective view of a camera main body 601 as viewed from the subject side, wherein a photographing lens unit has been removed. FIG. 16 is an exploded perspective view illustrating the schematic configuration of the inside of the camera for describing the peripheral structure of the dust removal device of the present invention and a photographing unit 400.

In the camera main body 601, a mirror box 605, to which a photographing luminous flux that has passed through a photographing lens is introduced, is provided, and a main mirror (quick-return mirror) 606 is disposed in the mirror box 605. The main mirror 606 can take a state in which the angle to the photographing optical axis is retained at 45° in order to introduce the photographing luminous flux to the direction of a penta-dach mirror (not shown) and a state in which the main mirror 606 is retained at a position deviated from the photographing luminous flux in order to introduce the photographing luminous flux to the direction of a photographing element (not shown).

On the subject side of a main body chassis 300 to serve as the backbone of the camera main body, the mirror box 605 and a shutter unit 200 are disposed in order from the subject side. On the photographer side of the main body chassis 300, the photographing unit 400 is disposed. The photographing unit 400 is set with the photographing face of the photographing element adjusted to lie at a specific distance from a mounting face of a mount section 602, as a reference for a photographing lens unit to be mounted, and lie in parallel to the mounting face.

Although a digital single-lens reflex camera has been described here as the imaging device of the present invention, the imaging device of the present invention may be a photographing lens unit-replaceable camera without including the mirror box 605 such as a mirror-less digital single-lens camera. The imaging device of the present invention can be applied to a photographing lens unite-replaceable video camera, various imaging apparatuses such as a copier, a facsimile and a scanner and an electronic device that includes an imaging device and especially needs removal of dust attached to the surface of an optical part. The photographing unit 400 can be applied as the imaging element unit of the present invention.

(Ultrasonic probe)

The ultrasonic probe of the present invention includes the piezoelectric element described above, wherein the ultrasonic probe transmits and receives signals by the piezoelectric element. By virtue of this configuration, an ultrasonic probe having a transmitting/receiving performance comparable to or higher than the transmitting/receiving performance in the case that a lead-containing piezoelectric element is used can be provided.

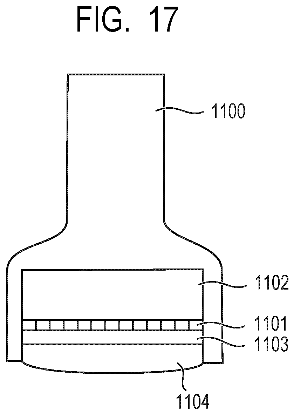

FIG. 17 is a schematic cross-sectional view illustrating a mode of the ultrasonic probe of the present invention.

An ultrasonic probe 1100 in FIG. 17 is configured with piezoelectric elements 1101, a backing material 1102, an acoustic matching layer 1103 and an acoustic lens 1104. As illustrated in FIG. 17, the plurality of piezoelectric elements 1101 is arrayed on and adhered to the backing material 1102, and the acoustic matching layer 1103 to match acoustic impedance is provided on the face on the opposite side to serve as a transmitting/receiving face.

The acoustic matching layer 1103 may be a monolayer or a multilayer, and can be of two or more layers. As the material for the acoustic matching layer 1103, for example, carbon, aluminum, aluminum alloy (e.g., Al-Mg alloy), magnesium alloy, Macor glass, glass, molten quartz, copper graphite, polyethylene, polypropylene, polycarbonate, ABC resin, polyphenylene ether, ABS resin, AAS resin, AES resin, nylon, polyamideimide, polyethylene terephthalate, polycarbonate, epoxy resin or urethane resin can be used.

For the backing material 1102, thermoplastic resin such as natural rubber, ferrite rubber, epoxy resin, polyvinyl chloride, polyvinyl butyral, ABS resin, polyurethane, polyvinyl alcohol, polyethylene, polypropylene, polyacetal, polyethylene terephthalate, fluororesin, polyethylene glycol and polyethylene terephthalate, or a mixture of any of the thermoplastic resins and metal powder, or a carbide material such as tungsten carbide can be used.

The piezoelectric elements 1101 may be integrated together or separated as a plurality of parts. FIG. 17 illustrates an example in which the piezoelectric elements 1101 are provided as separated parts. Flexible cables (not shown) are connected to the electrodes of each piezoelectric element, and signals transmitted and received by the piezoelectric elements can be inputted and outputted. The acoustic lens 1104 is adhered to the acoustic matching layer 1103. The acoustic lens 1104 is a member to converge ultrasonic waves transmitted from the piezoelectric elements 1101 to a subject, and is in the form of an arc in the example in FIG. 17. For the material of the acoustic lens 1104, for example, rubber containing silicone resin (rubber) as a main component is commonly used.

In using the ultrasonic probe 1100, alternating voltages are applied to the piezoelectric elements 1101 via flexible cables to vibrate the piezoelectric elements though the piezoelectric effect, thereby causing the piezoelectric elements 1101 to transmit ultrasonic waves. At that time, if the acoustic impedance of the subject on which the ultrasonic waves hit is small or ultrasonic waves are hit on the subject via water or air, the presence of the acoustic matching layer 1103 can prevent the generation of reflected waves due to large change in acoustic impedance, allowing the subject to be efficiently irradiated with ultrasonic waves. In reception, ultrasonic waves reflected in the inside of the subject vibrate piezoelectric elements 1101, and the vibration is electrically converted through the piezoelectric effect to obtain a reception signal. While conversion between electric energy and mechanical energy is performed in transmitting and receiving, the conversion efficiency at that time depends on the size of the electromechanical coupling factor.

(Ultrasonic diagnosis apparatus)

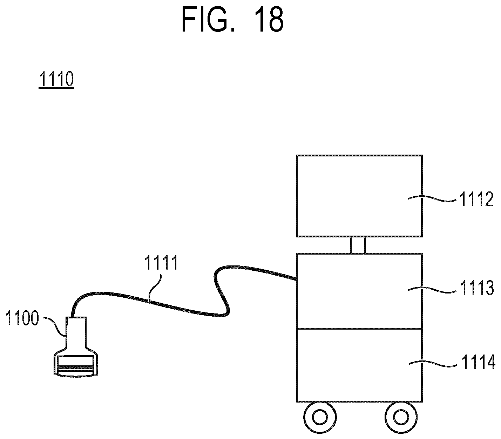

The ultrasonic diagnosis apparatus of the present invention at least includes the ultrasonic probe described above and an image output section (also referred to as an image display section). By virtue of this configuration, an ultrasonic diagnosis apparatus having a drive efficiency comparable to or higher than the drive efficiency in the case that a lead-containing piezoelectric element is used can be provided. FIG. 18 is a schematic diagram illustrating a mode of the ultrasonic diagnosis apparatus of the present invention. An ultrasonic diagnosis apparatus 1110 in FIG. 18 is configured with an ultrasonic probe 1100, a cable 1111, a drive control section 1113, an image display section 1112 and an image processing section 1114. The flexible cables of the ultrasonic probe 1100 and the drive control section 1113 are connected together via the cable 1111, and alternating voltages are applied to the piezoelectric elements 1101 of the ultrasonic probe 1100 from the drive control section 1113 via the cable 1111. When the inside of a subject is irradiated with ultrasonic waves from the ultrasonic probe 1100, ultrasonic waves reflected from the inside of the subject are converted again into electric signals in the ultrasonic probe 1100, and inputted into the image processing section 1114 through the cable 1111. The image processing section 1114 performs calculation with retardation time and signal intensity variation to alternating voltages outputted from the drive control section 1113 to generate an image data. The generated image data is outputted to the image display section 1112.

(Ultrasonic diagnosis system)

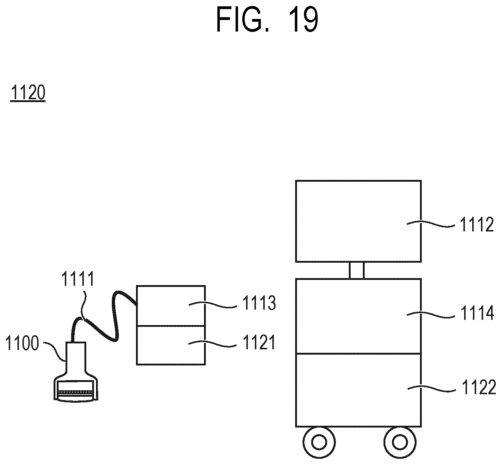

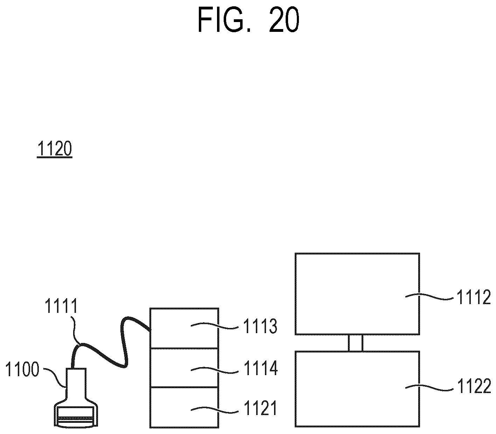

The ultrasonic diagnosis system of the present invention includes: the ultrasonic probe described above; a transmitting section that transmits signals outputted from the ultrasonic probe described above; and a receiving section that receives signals transmitted from the transmitting section. By virtue of this configuration, an ultrasonic diagnosis system having a drive efficiency comparable to or higher than the drive efficiency in the case that a lead-containing piezoelectric element is used can be provided. FIG. 19 and FIG. 20 are schematic diagrams illustrating a mode of the ultrasonic diagnosis system of the present invention. In an ultrasonic diagnosis system 1120 in FIG. 19, signals acquired by an ultrasonic probe 1100 are transmitted from a transmitting section 1121, and the signals are received by a receiving section 1122. An image processing section 1114 generates image data from the reception signal, and the image data is outputted to an image display section 1112. The transmitting section 1121 and the receiving section 1122 may be far apart from each other. Transmitting and receiving may be performed by means of radio communication such as Wi-Fi (R) and Bluetooth (R), or via network lines with connected network cables.

In an ultrasonic diagnosis system 1120 in FIG. 20, an image processing section 1114 generates an image data from outputted signals, the data is transmitted as signals from a transmitting section 1121, and the signals are received by a receiving section 1122. The reception signal is outputted to an image display section 1112.

(Electronic device)

Next, the electronic device of the present invention will be described. The electronic device of the present invention is provided with a piezoelectric acoustic part including the piezoelectric element described above or the laminate piezoelectric element described above. By virtue of this configuration, an electronic device having a sound generation performance comparable to or higher than the sound generation performance in the case that a lead-containing piezoelectric element is used can be provided. Piezoelectric acoustic parts include a speaker, a buzzer, a microphone, and a surface acoustic wave (SAW) element.

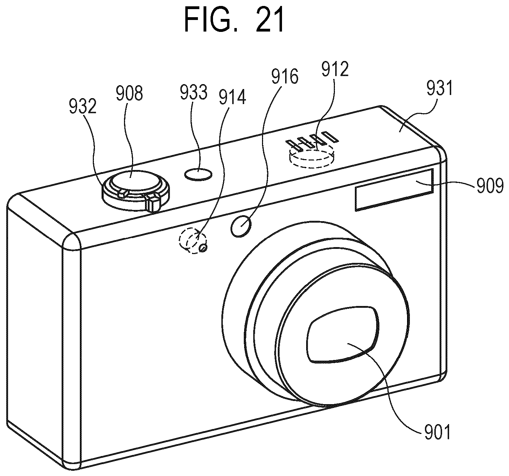

FIG. 21 is an overall perspective view of a main body 931 of a digital camera as an example of embodiments of the electronic device of the present invention as viewed from the front of the main body 931. On the front face of the main body 931, an optical device 901, a microphone 914, a strobe-light-emitting section 909 and a fill light section 916 are disposed. The microphone 914 is incorporated in the inside of the main body, and hence represented with dashed lines. In front of the microphone 914, a hole to pick up sounds from the exterior is provided.

On the top face of the main body 931, a power button 933, a speaker 912, a zoom lever 932 and a release button 908 to perform focusing operation are disposed. The speaker 912 is incorporated in the inside of the main body 931, and hence represented with dashed lines. In front of the speaker 912, a hole to transmit sounds to the exterior is provided.

The piezoelectric acoustic part of the present invention is used for at least one of the microphone 914, the speaker 912 and a surface audio wave element.

Although a digital camera has been described as the electronic device of the present invention, the electronic device of the present invention can also be applicable to various electronic devices including a piezoelectric acoustic part such as audio players, audio recorders, cell phones and information terminals.

As described above, the piezoelectric element of the present invention can be used for ultrasonic motors, optical devices, vibration devices, dust removal devices, imaging devices, ultrasonic probes, ultrasonic diagnosis apparatuses and electronic devices.

In addition to the devices described above, the single crystal and piezoelectric element of the present invention can be used for devices including motors, liquid-discharging heads, liquid-discharging devices, piezoelectric actuators, piezoelectric sensors and ferroelectric memories.

According to the present invention, there is provided a method for manufacturing a single crystal having a high electromechanical coupling factor and a high coercive field and the single crystal, wherein the single crystal is unproducible with any conventional production method.

Examples

Hereinafter, the present invention will be described in more detail with reference to examples, but the present invention is not limited by the following examples.

The single crystal and piezoelectric element of the present invention were produced by a method shown in the following.

(Example 1)

As raw material powders, barium titanate ($BaTiO_3$, Ba/Ti = 0. 999) having an average particle size of 100 nm, barium zirconate ($BaZrO_3$, Ba/Zr = 1.002), trimanganese tetraoxide ($Mn_3O_4$) and titanium oxide to adjust the ratio of the sum total of the numbers of moles of Ti and Zr to the sum total of the numbers of moles of Ba, a, were used. These raw material powders were weighed to obtain a ratio corresponding to the composition formula $Ba_{0.985}(Ti_{0.93},Zr_{0.07})O_3$ containing titanium and barium as main components. To 100 parts by mass of this oxide, Mn was added to yield a Mn content of 0.12 parts by mass in terms of metal. Further, the oxide to which Mn had been added was mixed by dry mixing with a ball mill for 24 hours. To granulate the resulting mixed powder, 3 parts by mass of a PVA binder to the amount of the mixed powder was attached to the surface of the mixed powder by using a spray dryer.

Next, a metal mold in which a face to be on the sample side had been mirror-finished was filled with the resulting granulated powder, and a molding pressure of 200 MPa was applied with a press molding machine to produce two cuboidal molded bodies. These molded bodies may be further pressurized by using a cold isometric press molding machine.

Next, one of the molded bodies was placed as a sample on a heating stage for microscopes (manufactured by Linkam Scientific Instruments Ltd.) and the temperature was increased, and microscopic observation was performed for the surface of the matrix 12 to find that the liquid-phase-appearing temperature was 1320°C and the liquid-phase-disappearing temperature was 1385°C.

Next, the other molded body was placed in an electric furnace and retained at the maximum temperature of 1300°C for 5 hours, and sintered under an atmospheric environment over 24 hours in total, yielding a 12 mm × 18 mm × 1.3 mm cuboidal matrix 12.

Next, each face of the obtained cuboidal matrix was polished by using a polisher with multiple abrasive grains differing in roughness to obtain a thickness of 1.0 mm, and finally subjected to chemical polishing as finishing to yield a mirror surface.

Next, a zirconia-coated alumina saggar as a saggar 17, a zirconia setter as a setter 16, a zirconia setter (size: 50 mm × 50 mm × 1 mm, weight: 20 g) as a weight 14 and zirconia beads as beads 15 were prepared, disposed as shown in FIG. 6, placed in an electric furnace with covering with a lid 18 as shown in FIG. 7, and fired.

The seed single crystal 11 used here was a 10 mm × 10 mm × 1 mm substrate of $BaTiO_3$ (manufactured by Physcience Opto-electronics Co., Ltd.) the principal surfaces and side faces of which were each a (100) plane and each face of which had been mirror-polished.

The temperature increase profile in firing was as follows: first temperature increase in which the temperature was increased from room temperature to 1320°C as the liquid-phase-appearing temperature at a temperature increase rate of 150°C/h over 8.8 hours was followed by second temperature increase in which the temperature was increased from 1320°C to 1385°C as the liquid-phase-disappearing temperature at a temperature increase rate of 0.65°C/h over 100 hours; thereafter, the temperature was retained at 1385°C for 300 h as firing; finally, the temperature was decreased to room temperature by letting the resultant to cool. The first temperature increase, second temperature increase and firing were performed in the atmosphere.

Observation of the fired matrix with an optical microscope confirmed that the single crystal growth part (the region spread from the seed single crystal) was a single crystal without any grain boundary.

Next, the seed single crystal 11 part was removed by polishing, and the single crystal part was cut out with a dicing saw, yielding a first single crystal.

Next, the crystal structure of the first single crystal was analyzed through X-ray diffraction. As a result of the analysis, only a peak corresponding to a perovskite-type (100)-oriented single crystal was observed.

Next, the first single crystal was placed on a stabilized zirconia setter 6 as shown in FIG. 2, the resultant was set in a high-temperature electric furnace of a reducing firing furnace illustrated in FIG. 1 and fired under a reducing environment at 1340°C for 5 hours, and the temperature was finally decreased to room temperature by letting the resultant to cool.

The atmosphere gas of the reducing environment used was argon containing 3 mol% of hydrogen in a gas cylinder, and the oxygen partial pressure immediately before firing was $5 \times 10^{-15}$ Pa.

Next, the crystal structure of the single crystal fired under a reducing environment was analyzed through X-ray diffraction. As a result of the analysis, a heterogenous phase as a crystal differing from perovskite was not observed. Next, the first single crystal 1 fired under a reducing environment was placed in a saggar 7 as shown in FIG. 3, the resultant was set on a stabilized zirconia setter 6, and the resultant was covered with a lid and placed in an electric furnace, and fired under an atmospheric environment at 1000°C for 10 hours, and the temperature was finally decreased to room temperature by letting the resultant to cool; thus, a single crystal was produced. In firing, the super electric furnace SSFT-1520 (manufactured by YAMADA DENKI CO., LTD.) was used.

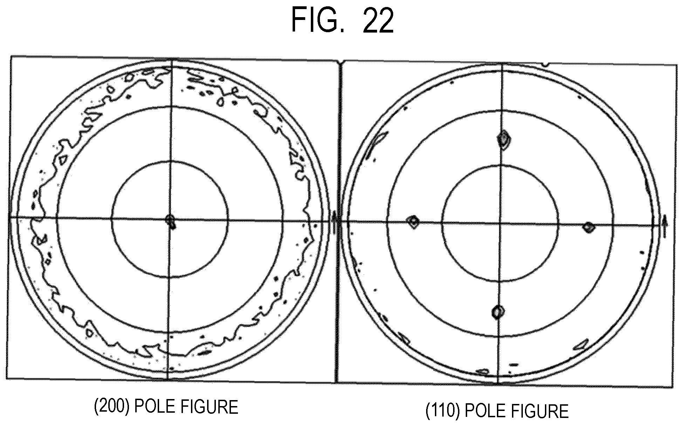

Next, the crystal structure of the produced single crystal was analyzed through X-ray diffraction. The result is shown in FIG. 22. From the (110) pole figure and the (200) pole figure, (100) orientation was observed. In addition, each of the side faces of the sample was a (200) plane.

Next, the composition of the single crystal was obtained through ICP emission spectrometry and assessed. The result is shown in Table 1. For Ba, Ti, Zr and Mn, the weighed composition and the composition after firing agreed with each other. Pb was not detected.

Next, the single crystal was processed into a 0.8 mm × 0.8 mm × 5.0 mm prism by polishing and dicing. At that time, processing was performed in such a manner that the crystal orientation of the 0.8 mm × 0.8 mm faces corresponded to (100).

Next, a gold electrode having a thickness of 400 nm was formed on each 0.8 mm × 0.8 mm face by a DC sputtering method; thus, the piezoelectric element of the present invention was produced.

Between each electrode and the single crystal, a 30-nm titanium film was formed as a close adhesion layer. Masking was performed for the 0.8 mm × 5.0 mm side faces in DC sputtering to avoid attachment of a gold electrode.

Next, the obtained piezoelectric element was subjected to polarization treatment by applying a 1 kV/mm electric field for 30 minutes on a hot plate set to have a surface temperature from 60°C to 100°C.

Next, the electromechanical coupling factor $k_{33}$ of the piezoelectric element including the single crystal of the present invention was assessed by a resonance-antiresonance method with an impedance analyzer (4294A manufactured by Agilent Technologies) at room temperature (25°C).

The result of assessment showed that the electromechanical coupling factor $k_{33}$ of this sample was 83%. The electromechanical coupling factor was found to be large for lead-free piezoelectric materials.

Next, measurement was performed every 5°C while the temperature of the sample was varied from 0°C to 70°C at a rate of 0.5°C per minute, and the variation rate of the piezoelectric constant d in the temperature range of 0°C or more and 70°C or less and the maximum value of the relative dielectric constant $\varepsilon_{33}$ were derived according to the following expressions (5) and (6):

$$d_{33} = k_{33} \sqrt{\frac{\varepsilon_{33}^T}{Y_{33}}} \qquad (5)$$

$$\text{Variation rate (\%) of } d_{33} = 100 \times (d_{33MAX} - d_{33MIN}) / d_{33@0°C} \quad (6)$$

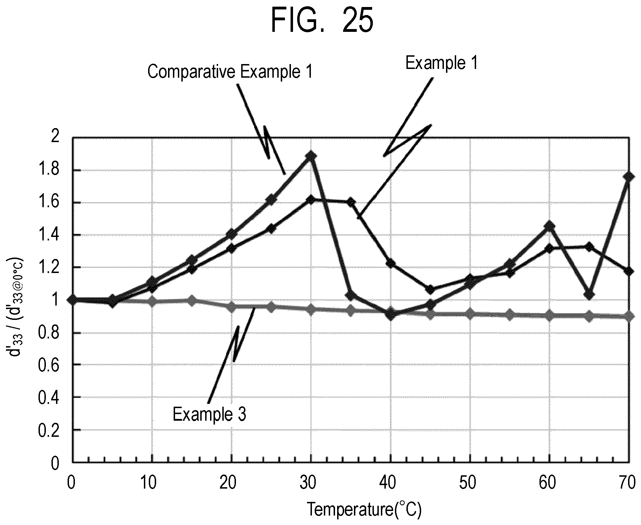

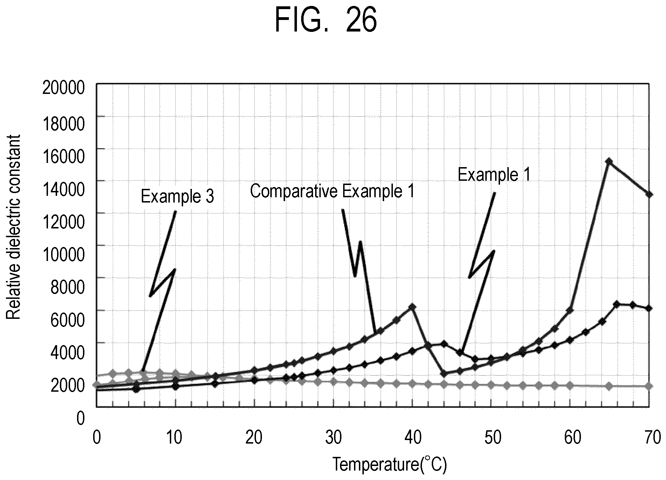

The results are shown in Table 1 and FIG. 25 and FIG. 26. Here, $\varepsilon^T$ denotes the dielectric constant, and the dielectric constant divided by the dielectric constant of vacuum corresponds to the relative dielectric constant.

The results found that the variation in $d_{33}$ was small and the maximum value of the relative dielectric constant $\varepsilon_{33}$ was small for single crystals.

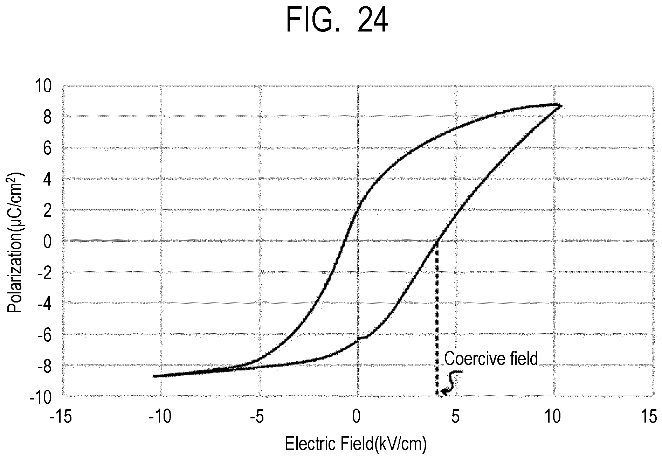

Next, the coercive field of the piezoelectric element was assessed. With use of a ferroelectrics/piezoelectrics assessment system (LC II manufactured by Radiant Technologies, Inc.), an electric field at a degree of polarization of 0 was read from a PE hysteresis curve given from degrees of polarization against electric fields in a hysteresis measurement mode in FIG. 24, and the value was regarded as the coercive field. The coercive field in this case was 4.0 kV/cm.

(Example 2)

The single crystal and piezoelectric element of the present invention were produced through the same process as in Example 1, except that the temperature for firing under a reducing environment was changed to 1385°C as shown in Table 1.

Subsequently, the composition of the single crystal of the present invention, the electromechanical coupling factor $k_{33}$, the coercive field, the maximum value of the relative dielectric constant at 0°C to 70°C and the variation rate of the piezoelectric constant were assessed through the same process as in Example 1. The results are shown in Table 1.

(Example 3)

As raw material powders, barium titanate ($BaTiO_3$, Ba/Ti = 0.999) having an average particle size of 100 nm, barium zirconate ($BaZrO_3$, Ba/Zr = 1.002), trimanganese tetraoxide ($Mn_3O_4$), bismuth oxide ($Bi_2O_3$), lithium carbonate ($Li_2CO_3$) and titanium oxide to adjust the ratio of the sum total of the numbers of moles of Ti and Zr to the sum total of the numbers of moles of Ba, a, were used. These raw material powders were weighed to obtain a ratio corresponding to the composition formula $Ba_{0.990}(Ti_{0.98},Zr_{0.02})O_3$ containing titanium and barium as main components.

To 100 parts by mass of this oxide, trimanganese tetraoxide ($Mn_3O_4$), bismuth oxide ($Bi_2O_3$) and lithium carbonate ($Li_2CO_3$) were added to yield a Mn content of 0.23 parts by mass in terms of metal, a Bi content of 0.18 parts by mass in terms of metal and a Li content of 0.15 parts by mass in terms of metal. Further, the oxide to which $Mn_3O_4$, $Bi_2O_3$ and $Li_2CO_3$ had been added was mixed by dry mixing with a ball mill for 24 hours. To granulate the resulting mixed powder, 3 parts by mass of a PVA binder to the amount of the mixed powder was attached to the surface of the mixed powder by using a spray dryer to form a granulated powder.

Next, a metal mold in which a face to be on the sample side had been mirror-finished was filled with the resulting granulated powder, and a molding pressure of 200 MPa was applied with a press molding machine to produce two cuboidal molded bodies. These molded bodies may be further pressurized by using a cold isometric press molding machine.

Next, one of the molded bodies was placed as a sample on a heating stage for microscopes (manufactured by Linkam Scientific Instruments Ltd.) and the temperature was increased, and microscopic observation was performed for the surface of the matrix 12 to find that the liquid-phase-appearing temperature was 1320°C and the liquid-phase-disappearing temperature was 1400°C.

Next, the other molded body was placed in an electric furnace and retained at the maximum temperature of 1300°C for 5 hours, and sintered under an atmospheric environment over 24 hours in total, yielding a 12 mm × 18 mm × 1.3 mm cuboidal matrix 12.

Next, each face of the obtained cuboidal matrix was polished by using a polisher with multiple abrasive grains differing in roughness to obtain a thickness of 1.0 mm, and finally subjected to chemical polishing as finishing to yield a mirror surface.

Next, a zirconia-coated alumina saggar as a saggar 17, a zirconia setter as a setter 16, a zirconia setter (size: 50 mm × 50 mm × 1 mm, weight: 20 g) as a weight 14 and zirconia beads as beads 15 were prepared, disposed as shown in FIG. 6, placed in an electric furnace with covering with a lid 18 as shown in FIG. 7, and fired.

The seed single crystal 11 used here was a 10 mm × 10 mm × 1 mm substrate of $BaTiO_3$ (manufactured by Physcience Opto-electronics Co., Ltd.) the principal surfaces and side faces of which were each a (110) plane and each face of which had been mirror-polished.

The temperature increase profile in firing was as follows: first temperature increase in which the temperature was increased from room temperature to 1320°C as the liquid-phase-appearing temperature at a temperature increase rate of 150°C/h over 8.8 hours was followed by second temperature increase in which the temperature was increased from 1320°C to 1400°C as the liquid-phase-disappearing temperature at a temperature increase rate of 0.80°C/h over 100 hours; thereafter, the temperature was retained at 1400°C for 1000 h as firing; finally, the temperature was decreased to room temperature by letting the resultant to cool. The first temperature increase, second temperature increase and firing were performed in the atmosphere.

Observation of the fired matrix with an optical microscope confirmed that the single crystal growth part (the region spread from the seed single crystal) was a single crystal without any grain boundary.

Next, the seed single crystal 11 part was removed by polishing, and the single crystal part was cut out with a dicing saw, yielding a first single crystal.

Next, the crystal structure of the first single crystal was analyzed through X-ray diffraction. As a result of the analysis, only a peak corresponding to a perovskite-type (110)-oriented single crystal was observed.

Next, the first single crystal was placed on a stabilized zirconia setter 6 as shown in FIG. 2, the resultant was set in a high-temperature electric furnace of a reducing firing furnace illustrated in FIG. 1 and fired under a reducing environment at 1280°C for 5 hours, and the temperature was finally decreased to room temperature by letting the resultant to cool.

The atmosphere gas of the reducing environment used was argon containing 3 mol% of hydrogen in a gas cylinder, and the oxygen partial pressure immediately before firing was $5 \times 10^{-10}$ Pa.

Next, the crystal structure of the single crystal fired under a reducing environment was analyzed through X-ray diffraction. As a result of the analysis, a heterogenous phase as a crystal differing from perovskite was not observed. Next, the first single crystal 1 fired under a reducing environment was placed in a saggar 7 as shown in FIG. 3, the resultant was set on a stabilized zirconia setter 6, and the resultant was covered with a lid and placed in an electric furnace, and fired under an atmospheric environment at 1000°C for 10 hours, and the temperature was finally decreased to room temperature by letting the resultant to cool; thus, a single crystal was produced. In firing, the super electric furnace SSFT-1520 (manufactured by YAMADA DENKI CO., LTD.) was used.

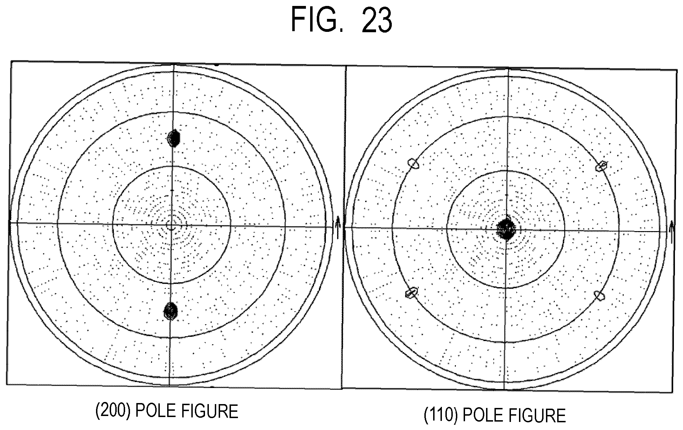

Next, the crystal structure of the produced single crystal was analyzed through X-ray diffraction. The result is shown in FIG. 23. From the (110) pole figure and the (200) pole figure, (110) orientation was observed.

Next, the composition of the single crystal was obtained through ICP emission spectrometry and evaluated. The result is shown in Table 1. For Ba, Ti, Zr, Mn and Bi, the weighed composition and the composition after firing agreed with each other. Pb was not detected.

Next, the single crystal was processed into a 0.8 mm × 0.8 mm × 5.0 mm prism by polishing and dicing. At that time, processing was performed in such a manner that the crystal orientation of the 0.8 mm × 0.8 mm faces corresponded to (110).

Next, a gold electrode having a thickness of 400 nm was formed on each 0.8 mm × 0.8 mm face by a DC sputtering method; thus, the piezoelectric element of the present invention was produced.

Between each electrode and the single crystal, a 30-nm titanium film was formed as a close adhesion layer. Masking was performed for the 0.8 mm × 5.0 mm side faces in DC sputtering to avoid attachment of a gold electrode.

Next, the obtained piezoelectric element was subjected to polarization treatment by applying a 1 kV/mm electric field for 30 minutes on a hot plate set to have a surface temperature from 60°C to 100°C.

Next, the electromechanical coupling factor $k_{33}$ of the piezoelectric element including the single crystal of the present invention was assessed by a resonance-antiresonance method with an impedance analyzer (4294A manufactured by Agilent Technologies) at room temperature (25°C).

The result of assessment showed that the electromechanical coupling factor $k_{33}$ of this sample was 82%. The electromechanical coupling factor was found to be large for lead-free piezoelectric materials.

Next, measurement was performed every 5°C while the temperature of the sample was varied from 0°C to 70°C at a rate of 0.5°C per minute, and the variation rate of the piezoelectric constant d in the temperature range of 0°C or more and 70°C or less and the maximum value of the relative dielectric constant $\varepsilon_{33}$ were derived according to the above expressions (5) and (6).

The results are shown in Table 1 and FIG. 25 and FIG. 26. Here, $\varepsilon^T$ denotes the dielectric constant, and the dielectric constant divided by the dielectric constant of vacuum corresponds to the relative dielectric constant.

The results found that the variation in $d_{33}$ was small and the maximum value of the relative dielectric constant $\varepsilon_{33}$ was small for single crystals.

Next, the coercive field of the piezoelectric element was assessed. With use of a ferroelectrics/piezoelectrics assessment system (LC II manufactured by Radiant Technologies, Inc.), an electric field at a degree of polarization of 0 was read from a PE hysteresis curve given from degrees of polarization against electric fields in a hysteresis measurement mode in FIG. 24, and the value was regarded as the coercive field. The coercive field in this case was 7.5 kV/cm.

(Comparative Example 1)

As raw material powders, barium titanate ($BaTiO_3$, Ba/Ti = 0.999) having an average particle size of 100 nm, barium zirconate ($BaZrO_3$, Ba/Zr = 1.002), trimanganese tetraoxide ($Mn_3O_4$) and titanium oxide to adjust the ratio of the sum total of the numbers of moles of Ti and Zr to the sum total of the numbers of moles of Ba, a, were used. These raw material powders were weighed to obtain a ratio corresponding to the composition formula $Ba_{0.985}(Ti_{0.93},Zr_{0.07})O_3$ containing titanium and barium as main components. To 100 parts by mass of this oxide, Mn was added to yield a Mn content of 0.12 parts by mass in terms of metal. Further, the oxide to which Mn had been added was mixed by dry mixing with a ball mill for 24 hours. To granulate the resulting mixed powder, 3 parts by mass of a PVA binder to the amount of the mixed powder was attached to the surface of the mixed powder by using a spray dryer.

Next, a metal mold in which a face to be on the sample side had been mirror-finished was filled with the resulting granulated powder, and a molding pressure of 200 MPa was applied with a press molding machine to produce two cuboidal molded bodies.

Next, one of the molded bodies was placed as a sample on a heating stage for microscopes (manufactured by Linkam Scientific Instruments Ltd.) and the temperature was increased, and microscopic observation was performed for the surface of the matrix 12 to find that the liquid-phase-appearing temperature was 1320°C and the liquid-phase-disappearing temperature was 1385°C.

Next, the other molded body was placed in an electric furnace and retained at the maximum temperature of 1300°C for 5 hours, and sintered under an atmospheric environment over 24 hours in total, yielding a 12 mm × 18 mm × 1.3 mm cuboidal matrix 12.

Next, each face of the obtained cuboidal matrix 12 was polished by using a polisher with multiple abrasive grains differing in roughness to obtain a thickness of 1.0 mm, and finally subjected to chemical polishing as finishing to yield a mirror surface.

Next, a zirconia-coated alumina saggar as a saggar 17, a zirconia setter as a setter 16, a zirconia setter (size: 50 mm × 50 mm × 1 mm, weight: 20 g) as a weight 14 and zirconia beads as beads 15 were prepared, disposed as shown in FIG. 6, placed in an electric furnace with covering with a lid as shown in FIG. 7, and fired.

The seed single crystal 11 used here was a 10 mm × 10 mm × 1 mm substrate of $BaTiO_3$ (manufactured by Physcience Opto-electronics Co., Ltd.) the top and bottom faces and side faces of which were each a (100) plane and each face of which had been mirror-polished.

The temperature increase profile in firing was as follows: first temperature increase in which the temperature was increased from room temperature to 1320°C as the liquid-phase-appearing temperature at a temperature increase rate of 150°C/h over 8.8 hours was followed by second temperature increase in which the temperature was increased from 1320°C to 1385°C as the liquid-phase-disappearing temperature at a temperature increase rate of 0.65°C/h over 100 hours; thereafter, the temperature was retained at 1385°C for 300 h as firing; finally, the temperature was decreased to room temperature by letting the resultant to cool. The first temperature increase, second temperature increase and firing were performed in the atmosphere.

Observation of the fired matrix 12 with an optical microscope confirmed that the single crystal growth part (the region spread from the seed single crystal) was a single crystal without any grain boundary.

Next, the seed single crystal 11 part was removed by polishing, and the single crystal part was cut out with a dicing saw, yielding a single crystal of Comparative Example 1.

Next, the crystal structure of the produced single crystal was analyzed through X-ray diffraction. From the (110) pole figure and the (200) pole figure, (100) orientation was observed. In addition, each of the side faces of the sample was a (200) plane.

Next, the composition of the single crystal was obtained through ICP emission spectrometry and evaluated. The result is shown in Table 1. For Ba, Ti, Zr and Mn, the weighed composition and the composition after firing agreed with each other. Pb was not detected.

Next, the single crystal was processed into a 0.8 mm × 0.8 mm × 5.0 mm prism by polishing and dicing. At that time, processing was performed in such a manner that the crystal orientation of the 0.8 mm × 0.8 mm faces corresponded to (100).

Next, a gold electrode having a thickness of 400 nm was formed on each 0.8 mm × 0.8 mm face by a DC sputtering method; thus, a piezoelectric element of Comparative Example 1 was produced.

Between each electrode and the single crystal, a 30-nm titanium film was formed as a close adhesion layer. Masking was performed for the 0.8 mm × 5.0 mm side faces in DC sputtering to avoid attachment of a gold electrode.

Next, the obtained piezoelectric element was subjected to polarization treatment by applying a 1 kV/mm electric field for 30 minutes on a hot plate set to have a surface temperature from 60°C to 100°C.

Next, the electromechanical coupling factor $k_{33}$ of the piezoelectric element including the single crystal of Comparative Example 1 was assessed by a resonance-antiresonance method with an impedance analyzer (4294A manufactured by Agilent Technologies) at room temperature (25°C).

The result of assessment showed that the $k_{33}$ of this sample was 79%.

Next, measurement was performed every 5°C while the temperature of the sample was varied from 0°C to 70°C at a rate of 0.5°C per minute, and the variation rate of the piezoelectric constant $d_{33}$ in the temperature range of 0°C or more and 70°C or less and the maximum value of the relative dielectric constant $\varepsilon_{33}$ were derived according to the above expressions (5) and (6).

The results are shown in Table 1. Here, $\varepsilon^T$ denotes the dielectric constant, and the dielectric constant divided by the dielectric constant of vacuum corresponds to the relative dielectric constant.

Next, the coercive field of the piezoelectric element was assessed. With use of a ferroelectrics/piezoelectrics assessment system (LC II manufactured by Radiant Technologies, Inc.), an electric field at a degree of polarization of 0 was read from a PE hysteresis curve given from degrees of polarization against electric fields, and the value was regarded as the coercive field. The coercive field in this case was 1.6 kV/cm.

(Comparative Example 2)

As raw material powders, barium titanate ($BaTiO_3$, Ba/Ti = 0.999) having an average particle size of 100 nm, barium zirconate ($BaZrO_3$, Ba/Zr = 1.002), trimanganese tetraoxide ($Mn_3O_4$), bismuth oxide ($Bi_2O_3$), lithium carbonate ($Li_2CO_3$) and titanium oxide to adjust the ratio of the sum total of the numbers of moles of Ti and Zr to the sum total of the numbers of moles of Ba, a, were used. These raw material powders were weighed to obtain a ratio corresponding to the composition formula $Ba_{0.990}(Ti_{0.98},Zr_{0.02})O_3$ containing titanium and barium as main components.

To 100 parts by mass of this oxide, trimanganese tetraoxide ($Mn_3O_4$), bismuth oxide ($Bi_2O_3$) and lithium carbonate ($Li_2CO_3$) were added to yield a Mn content of 0.23 parts by mass in terms of metal, a Bi content of 0.18 parts by mass in terms of metal and a Li content of 0.15 parts by mass in terms of metal. Further, the oxide to which $Mn_3O_4$, $Bi_2O_3$ and $Li_2CO_3$ had been added was mixed by dry mixing with a ball mill for 24 hours. To granulate the resulting mixed powder, 3 parts by mass of a PVA binder to the amount of the mixed powder was attached to the surface of the mixed powder by using a spray dryer to form a granulated powder.

Next, a metal mold in which a face to be on the sample side had been mirror-finished was filled with the resulting granulated powder, and a molding pressure of 200 MPa was applied with a press molding machine to produce two cuboidal molded bodies.

Next, one of the molded bodies was placed as a sample on a heating stage for microscopes (manufactured by Linkam Scientific Instruments Ltd.) and the temperature was increased, and microscopic observation was performed for the surface of the matrix 12 to find that the liquid-phase-appearing temperature was 1320°C and the liquid-phase-disappearing temperature was 1400°C.

Next, the other molded body was placed in an electric furnace and retained at the maximum temperature of 1300°C for 5 hours, and sintered under an atmospheric environment over 24 hours in total, yielding a 12 mm × 18 mm × 1.3 mm cuboidal matrix 12.

Next, each face of the obtained cuboidal matrix 12 was polished by using a polisher with multiple abrasive grains differing in roughness to obtain a thickness of 1.0 mm, and finally subjected to chemical polishing as finishing to yield a mirror surface.

Next, a zirconia-coated alumina saggar as a saggar 17, a zirconia setter as a setter 16, a zirconia setter (size: 50 mm × 50 mm × 1 mm, weight: 20 g) as a weight 14 and zirconia beads as beads 15 were prepared, disposed as shown in FIG. 6, placed in an electric furnace with covering with a lid as shown in FIG. 7, and fired.

The seed single crystal 11 used here was a 10 mm × 10 mm × 1 mm substrate of $BaTiO_3$ (manufactured by Physcience Opto-electronics Co., Ltd.) the top and bottom faces and side faces of which were each a (100) plane and each face of which had been mirror-polished.

The temperature increase profile in firing was as follows: first temperature increase in which the temperature was increased from room temperature to 1320°C as the liquid-phase-appearing temperature at a temperature increase rate of 150°C/h over 8.8 hours was followed by second temperature increase in which the temperature was increased from 1320°C to 1400°C as the liquid-phase-disappearing temperature at a temperature increase rate of 0.80°C/h over 100 hours; thereafter, the temperature was retained at 1400°C for 1000 h as firing; finally, the temperature was decreased to room temperature by letting the resultant to cool. The first temperature increase, second temperature increase and firing were performed in the atmosphere.

Observation of the fired matrix 12 with an optical microscope confirmed that the single crystal growth part (the region spread from the seed single crystal) was a single crystal without any grain boundary.

Next, the seed single crystal 11 part was removed by polishing, and the single crystal part was cut out with a dicing saw, yielding a single crystal of Comparative Example 2.

Next, the crystal structure of the produced single crystal was analyzed through X-ray diffraction. From the (110) pole figure and the (200) pole figure, (110) orientation was observed.

Next, the composition of the single crystal was obtained through ICP emission spectrometry and evaluated. The result is shown in Table 1. For Ba, Ti, Zr, Mn and Bi, the weighed composition and the composition after firing agreed with each other. Pb was not detected.

Next, the single crystal was processed into a 0.8 mm × 0.8 mm × 5.0 mm prism by polishing and dicing. At that time, processing was performed in such a manner that the crystal orientation of the 0.8 mm × 0.8 mm faces corresponded to (110).

Next, a gold electrode having a thickness of 400 nm was formed on each 0.8 mm × 0.8 mm face by a DC sputtering method; thus, a piezoelectric element of Comparative Example 2 was produced.

Between each electrode and the single crystal, a 30-nm titanium film was formed as a close adhesion layer. Masking was performed for the 0.8 mm × 5.0 mm side faces in DC sputtering to avoid attachment of a gold electrode.

Next, the obtained piezoelectric element was subjected to polarization treatment by applying a 1 kV/mm electric field for 30 minutes on a hot plate set to have a surface temperature from 60°C to 100°C.

Next, the electromechanical coupling factor $k_{33}$ of the piezoelectric element including the single crystal of Comparative Example 2 was assessed by a resonance-antiresonance method with an impedance analyzer (4294A manufactured by Agilent Technologies) at room temperature (25°C).

The result of assessment showed that the $k_{33}$ of this sample was 75%.

Next, measurement was performed every 5°C while the temperature of the sample was varied from 0°C to 70°C at a rate of 0.5°C per minute, and the variation rate of the piezoelectric constant $d_{33}$ in the temperature range of 0°C or more and 70°C or less and the maximum value of the relative dielectric constant $\varepsilon_{33}$ were derived according to the above expressions (5) and (6).

The results are shown in Table 1. Here, $\varepsilon^T$ denotes the dielectric constant, and the dielectric constant divided by the dielectric constant of vacuum corresponds to the relative dielectric constant.

Next, the coercive field of the piezoelectric element was assessed. With use of a ferroelectrics/piezoelectrics assessment system (LC II manufactured by Radiant Technologies, Inc.), an electric field at a degree of polarization of 0 was read from a PE hysteresis curve given from degrees of polarization against electric fields, and the value was regarded as the coercive field. The coercive field in this case was 5.6 kV/cm.

[Table 1]

Table 1

| | Composition of single crystal | Firing temperature (°C) for first single crystal | Firing temperature (°C) under reducing environment | Firing temperature (°C) under atmospheric environment | Electromechanical coupling factor $k_{33}$ (%) |
|---|---|---|---|---|---|
| Example 1 | $Ba_{0.985}(Ti_{0.93},Zr_{0.07})O_3$ + Mn 0.12% by mass | 1385 | 1340 | 1000 | 85 |
| Example 2 | $Ba_{0.985}(Ti_{0.93},Zr_{0.07})O_3$ + Mn 0.12% by mass | 1385 | 1385 | 1000 | 83 |
| Example 3 | $Ba_{0.990}(Ti_{0.98},Zr_{0.02})O_3$ + Mn 0.23% by mass + Bi 0.18% by mass | 1400 | 1280 | 1000 | 82 |
| Comparative Example 1 | $Ba_{0.985}(Ti_{0.93},Zr_{0.07})O_3$ + Mn 0.12% by mass | 1385 | - | - | 79 |
| Comparative Example 2 | $Ba_{0.990}(Ti_{0.98},Zr_{0.02})O_3$ + Mn 0.23% by mass + Bi 0.18% by mass | 1400 | - | - | 75 |
| | Composition of single crystal | Coercive field Ec (kV/cm) | Variation rate (%) of piezoelectric constant $d_{33}$ @ 0 to 70°C | Maximum value of relative dielectric constant @ 0 to 70°C | Maximum value of relative dielectric constant @ 0 to 60°C |
| Example 1 | $Ba_{0.985}(Ti_{0.93},Zr_{0.07})O_3$ + Mn 0.12% by mass | 4.0 | 61 | 6300 | 4000 |
| Example 2 | $Ba_{0.985}(Ti_{0.93},Zr_{0.07})O_3$ + Mn 0.12% by mass | 2.5 | 63 | 6500 | 4200 |
| Example 3 | $Ba_{0.990}(Ti_{0.98},Zr_{0.02})O_3$ + Mn 0.23% by mass + Bi 0.18% by mass | 7.5 | 10 | 1900 | 1900 |
| Comparative Example 1 | $Ba_{0.985}(Ti_{0.93},Zr_{0.07})O_3$ + Mn 0.12% by mass | 1.6 | 98 | 15100 | 6200 |
| Comparative Example 2 | $Ba_{0.990}(Ti_{0.98},Zr_{0.02})O_3$ + Mn 0.23% by mass + Bi 0.18% by mass | 5.6 | 25 | 3200 | 3200 |

(Example 4)

The ultrasonic motor illustrated in FIG. 9 was produced with the piezoelectric element of Example 1. In the ultrasonic motor produced, the rotation of the motor in response to application of an alternating voltage was confirmed.

(Example 5)

The optical device illustrated in FIG. 11 was produced with the ultrasonic motor of Example 4. In the optical device produced, autofocus operation in response to application of an alternating voltage was confirmed.

(Example 6)

The dust removal device illustrated in FIG. 12A and FIG. 12B was produced with the piezoelectric element of Example 1. When plastic beads were scattered and an alternating voltage was applied to the dust removal device produced, a good dust removal rate was confirmed.

(Example 7)

The imaging device illustrated in FIG. 15 was produced with the dust removal device of Example 6. When the imaging device produced was operated, dust on the surface of the photographing unit was well removed, and an image without any dust defect was obtained.

(Example 8)

The ultrasonic probe illustrated in FIG. 17 was produced with the piezoelectric element of Example 1. In the ultrasonic probe produced, ultrasonic waves were transmitted through application of an alternating voltage, and reception signals derived from reflection in the inside of a subject were confirmed.

(Example 9)

The ultrasonic diagnosis apparatus illustrated in FIG. 18 was produced with the ultrasonic probe of Example 8. When the ultrasonic diagnosis apparatus produced was operated, an image of the inside of a subject was clearly outputted.

(Example 10)

The ultrasonic diagnosis system illustrated in FIG. 19 was produced with the ultrasonic probe of Example 8. When the ultrasonic diagnosis system produced was operated to display on the image display section, an image of the inside of a subject was clearly outputted.

(Example 11)

The electronic device illustrated in FIG. 21 was produced with the piezoelectric element of Example 1. In the electronic device produced, a speaker operation in response to application of an alternating voltage was confirmed.

The single crystal of the present invention has an electromechanical coupling factor and a high coercive field. Accordingly, the single crystal of the present invention can be used for a number of devices including ultrasonic motors, dust removal devices and ultrasonic probes without causing any problem.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1. A method for manufacturing a perovskite-type single crystal, comprising:

   step (1): firing a raw material comprising an acceptor under an atmospheric environment to produce a first single crystal of perovskite type,
   step (2): firing the first single crystal under a reducing environment, and
   step (3): firing the single crystal produced in step (2) under an atmospheric environment;

   wherein, when performed in this order, a single crystal having a higher coercive field value than the first single crystal is produced.

2. The method for manufacturing a perovskite-type single crystal according to claim 1, wherein the acceptor is Mn.

3. The method for manufacturing a perovskite-type single crystal according to claim 1 or 2, wherein a firing temperature for firing under a reducing environment in step (2) is equal to or lower than a firing temperature for producing a first single crystal in step (1).

4. The method for manufacturing a perovskite-type single crystal according to any one of claims 1 to 3, wherein a firing temperature for firing under an atmospheric environment in step (3) is equal to or lower than a firing temperature for firing under a reducing environment in step (2).

5. The method for manufacturing a perovskite-type single crystal according to any one of claims 1 to 4, wherein the raw material comprises Ba, Ti and Zr.

6. The method for manufacturing a perovskite-type single crystal according to any one of claims 1 to 5, wherein producing a first single crystal in step (1) is by a solid-phase growth method.

7. The method for manufacturing a perovskite-type single crystal according to any one of claims 1 to 6, wherein the raw material comprises an oxide comprising Ba, Ti and Zr, and Mn, with x as a mole ratio of Zr to sum total of Ti and Zr satisfying $0.02 \leq x \leq 0.13$, and has a Mn content of 0.04 parts by mass or more and 0.36 parts by mass or less in terms of metal to 100 parts by mass of the oxide.

8. The method for manufacturing a perovskite-type single crystal according to any one of claims 1 to 6, wherein the raw material comprises an oxide comprising Ba, Ti and Zr, and Mn, Bi and Li, with x as a mole ratio of Zr to sum total of Ti and Zr satisfying $0.02 \leq x \leq 0.13$, and has a Mn content of 0.04 parts by mass or more and 0.36 parts by mass or less in terms of metal to 100 parts by mass of the oxide, a Bi content of more than 0 parts by mass and 0.20 parts by mass or less in terms of metal to 100 parts by mass of the oxide, and a Li content of 0.05 parts by mass or more and 0.20 parts by mass or less in terms of metal to 100 parts by mass of the oxide.

9. The method for manufacturing a perovskite-type single crystal according to any one of claims 1 to 8, wherein the reducing environment in step (2) is a gas comprising argon and hydrogen, and has an oxygen partial pressure $P(O_2)$ of $1 \times 10^{-10}$ Pa or less.

10. The method for manufacturing a perovskite-type single crystal according to any one of claims 1 to 9, wherein a firing temperature for firing under a reducing environment in step (2) is 1280°C or more.

11. The method for manufacturing a perovskite-type single crystal according to any one of claims 1 to 10, wherein a firing temperature for firing under an atmospheric environment in step (3) is 1000°C or less.

12. A perovskite-type single crystal comprising an oxide comprising Ba, Ti and Zr with perovskite structure, and Mn, with x as a mole ratio of Zr to sum total of Ti and Zr satisfying $0.02 \leq x \leq 0.13$, wherein

the single crystal has a Mn content of 0.04 parts by mass or more and 0.36 parts by mass or less in terms of metal to 100 parts by mass of the oxide,
the single crystal optionally comprises Bi, and has a Bi content of 0 parts by mass or more and 0.20 parts by mass or less in terms of metal to 100 parts by mass of the oxide, and
the single crystal has an electromechanical coupling factor $k_{33}$ of 80% or more at 25°C and a coercive field of 2.5 kV/cm or more.

13. The perovskite-type single crystal according to claim 12, wherein the single crystal comprises less than 1000 ppm of a Pb component.

14. The perovskite-type single crystal according to claim 12 or 13, wherein a variation width (%) of a piezoelectric constant $d_{33}$ of the single crystal is 65% or less, the variation width being represented by the following expression (1):

$$100 \times \Delta d_{33@0°C \text{ to } 70°C} / d_{33@0°C} \quad (1).$$

15. The perovskite-type single crystal according to any one of claims 12 to 14, wherein a maximum value of a relative dielectric constant $\varepsilon_{33}$ of the single crystal in a range of 0°C or more and 70°C or less is 6500 or less.

16. The perovskite-type single crystal according to claim 15, wherein a maximum value of a relative dielectric constant $\varepsilon_{33}$ of the single crystal in a range of 0°C or more and 60°C or less is 4200 or less.

17. A piezoelectric element comprising a plurality of electrodes and a single crystal, wherein the single crystal is the single crystal according to any one of claims 12 to 16.

18. An ultrasonic motor at least comprising: a vibration unit provided with the piezoelectric element according to claim 17;

and a mobile unit in contact with the vibration unit.

19. An optical device comprising the ultrasonic motor according to claim 18 in a drive section.

20. A vibration device comprising a vibration unit with a diaphragm provided with the piezoelectric element according to claim 17.

21. A dust removal device comprising the vibration device according to claim 20 in a vibration section.

22. An imaging device at least comprising the dust removal device according to claim 21 and an imaging element unit, wherein the diaphragm of the dust removal device is provided on a light-receiving-face side of the imaging element unit.

23. An ultrasonic probe comprising the piezoelectric element according to claim 17, wherein the ultrasonic probe transmits and receives an ultrasonic wave by the piezoelectric element.

24. An ultrasonic diagnosis apparatus at least comprising the ultrasonic probe according to claim 23 and an image output section.

25. An ultrasonic diagnosis system comprising: the ultrasonic probe according to claim 23; a transmitting section that transmits a signal outputted from the ultrasonic probe; and a receiving section that receives a signal transmitted from the transmitting section.

26. An electronic device provided with a piezoelectric acoustic part comprising the piezoelectric element according to claim 17.

FIG. 1

# FIG. 2

FIG. 3

FIG. 4

# FIG. 5

FIG. 6

# FIG. 7

# FIG. 8

FIG. 9

# FIG. 10A

# FIG. 10B

# FIG. 11

## FIG. 12A

310

320

333 332 331

330

## FIG. 12B

310

330

320

332

# FIG. 13A

# FIG. 13B

# FIG. 13C

# FIG. 14A

310

IN-PHASE

FIXED          320          FIXED

FIXED                       FIXED

330

# FIG. 14B

FIXED

REVERSE PHASE          FIXED          320

FIXED

FIXED

310

# FIG. 15

# FIG. 16

605

200

300

400

## FIG. 17

# FIG. 18

1110

# FIG. 19

1120

# FIG. 20

1120

1111

1100

1113

1114

1121

1112

1122

# FIG. 21

# FIG. 22

(200) POLE FIGURE

(110) POLE FIGURE

# FIG. 23

(200) POLE FIGURE         (110) POLE FIGURE

# FIG. 24

# FIG. 25

FIG. 26

Europäisches
Patentamt
European
Patent Office
Office européen
des brevets

# EUROPEAN SEARCH REPORT

**Application Number**

EP 25 17 1581

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PASKIEWICZ DEBORAH M. ET AL: "Single-Crystalline SrRuO 3 Nanomembranes: A Platform for Flexible Oxide Electronics", NANO LETTERS, vol. 16, no. 1, 16 December 2015 (2015-12-16), pages 534-542, XP093303283, ISSN: 1530-6984, DOI: 10.1021/acs.nanolett.5b04176 | 1,3,4,9, 11 | INV. C30B1/02 C30B29/32 C04B35/468 H10N30/00 C30B33/02 |
| A | * abstract * * figure 4b * * page 538, right-hand column, lines 1-13 * | 2,5-8,10 | |
| A | US 2023/329120 A1 (LEE HO YONG [KR] ET AL) 12 October 2023 (2023-10-12) * claims 1, 4, 14 * | 12-26 | |
| A | US 2022/293849 A1 (MATSUDA TAKANORI [JP] ET AL) 15 September 2022 (2022-09-15) * tables 1, 2 * | 12-26 | |

| TECHNICAL FIELDS SEARCHED (IPC) |
|---|
| C30B H02N C04B H10N |

The present search report has been drawn up for all claims

| Place of search | Date of completion of the search | Examiner |
|---|---|---|
| The Hague | 7 August 2025 | Schmitt, Christian |

EPO FORM 1503 03.82 (P04C01)

EP 4 640 929 A1

## ANNEX TO THE EUROPEAN SEARCH REPORT
## ON EUROPEAN PATENT APPLICATION NO.

EP 25 17 1581

07-08-2025

| Patent document cited in search report | Publication date | Patent family member(s) | | Publication date |
|---|---|---|---|---|
| US 2023329120 A1 | 12-10-2023 | JP | 7629656 B2 | 14-02-2025 |
| | | JP | 2023553068 A | 20-12-2023 |
| | | US | 2023329120 A1 | 12-10-2023 |
| | | WO | 2022124794 A1 | 16-06-2022 |
| US 2022293849 A1 | 15-09-2022 | JP | 7629761 B2 | 14-02-2025 |
| | | JP | 2022139322 A | 26-09-2022 |
| | | US | 2022293849 A1 | 15-09-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3507821 B

**Non-patent literature cited in the description**

- Iwanami Rikagaku Jiten. Iwanami Shoten, Publishers, 20 February 1998